(19) Europäisches Patentamt
European Patent Office
Office européen des brevets

(11) Publication number: 0 043 630 B1

(12) # EUROPEAN PATENT SPECIFICATION

(45) Date of publication of patent specification: 08.01.86

(51) Int. Cl.$^4$: C 07 F 7/08, C 07 F 7/10, C 07 F 7/18

(21) Application number: 81200771.4

(22) Date of filing: 03.07.81

(54) **Improved process for the silylation of organic compounds with 1,1,1-trimethyl-N-(trimethylsilyl)silanamine by means of catalysis with certain nitrogen containing compounds.**

(30) Priority: 04.07.80 NL 8003891
05.09.80 NL 8005041

(43) Date of publication of application:
13.01.82 Bulletin 82/02

(45) Publication of the grant of the patent:
08.01.86 Bulletin 86/02

(84) Designated Contracting States:
AT BE CH DE FR GB IT LI LU NL SE

(56) References cited:
DE-A-1 959 523
DE-A-2 757 936
GB-A-1 509 691

Journal of Organometallic Chemistry, vol. 61, pages 83-90, 1973

The file contains technical information submitted after the application was filed and not included in this specification

(73) Proprietor: Gist - Brocades N.V.
Wateringseweg 1 P.O. Box 1
NL-2600 MA Delft (NL)

(72) Inventor: Bruynes, Cornelis Adrianus
Ridderhoflaan 51
NL-2396 CK Koudekerk a/d Rijn (NL)
Inventor: Jurriens, Theodorus Klaas
Van der Haertstraat 43
NL-2613 ZA Delft (NL)

(74) Representative: Van der Straaten, Jan Anthony et al.
c/o GIST-BROCADES N.V. Patents and Trademarks Department Wateringseweg 1 P.O. Box 1
NL-2600 MA Delft (NL)

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

EP 0 043 630 B1

**Description**

The invention relates to an improved process for the trimethylsilylation of organic compounds.

In preparative organic chemistry there is a growing interest in the use of the trimethylsilyl group, as well for the protection of reactive groups, as for the modification of physical properties, such as volatility and solubility (see for example B. E. Cooper, Chem. and Ind. *1978*, 794).

Silylating agents which are used on a large scale are, for example, trimethylchlorosilane and dimethyldichlorosilane. Due to the fact that silylation reactions are equilibrium reactions, it is essential to remove the hydrogen chloride, which is generated in the reaction, as soon as possible, in order to shift the equilibrium to the side of the products. This can be done by the addition of a suitable tertiary amine to the reaction mixture. The amine reacts with the hydrogen chloride under the formation of the corresponding ammonium salt, which is usually poorly soluble in the reaction mixture. The removal of this ammonium salt is usually necessary before the product can be purified, which necessitates the use of large amounts of a suitable solvent. However, it is often unavoidable that traces of ammonium salt remain in the product.

Other silylating agents which are frequently used, e.g. N,O - (bis - trimethylsilyl)acetamide, N,N' - bis(trimethylsilyl)urea, N - trimethylsilyl - N,N' - diphenylurea, N,N - bis(trimethylsilyl)hydantoins, N - trimethylsilylimidazole and tri - methylsilyldiethylamine, which compounds often are prepared starting from trimethylchlorosilane, have the disadvantage that the silylated product has to be separated from the remainder, which is left from the silylating agent. For example, according to British Patent No. 1,509,691, in the silylation of e.g. sterically hindered phenols, bis(trimethylsilyl)hydantoins proved to be more effective than trimethylsilyldiethylamine, hexamethyldisilazane, and hexamethyldisilazane catalyzed by trimethylchlorosilane or concentrated sulphuric acid.

Another silylating agent which is used on a large scale is 1,1,1 - trimethyl - N - (trimethylsilyl)-silanamine (which is known under its trivial name hexamethyldisilazane, HMDS), which has the advantage that the only by-product is the gaseous, and therefore easily removable ammonia. Furthermore, HMDS is a relatively cheap reagent, which makes it attractive for industrial processes.

However, an important disadvantage of HMDS is that it reacts slowly in many instances, and in some cases it even does not react at all (see e.g. S. H. Langer c.s., J. Org. Chem. *23*, 50 (1958)). Consequently, high reaction temperatures and/or long reaction times are necessary to complete the silylation, which makes the method less attractive and unsuitable for heat-sensitive compounds. Furthermore, a large excess of HMDS is often required.

Therefore, much attention has been paid to the catalysis of silylation reactions with HMDS in order to lower the reaction temperature and/or to shorten the reaction time. Examples of catalysts are amine salts (see e.g. DE—A—2507882), trimethylchlorosilane (see e.g. S. H. Langer c.s., J. Org. Chem. *23*, 50 (1958)), inorganic acids such as sulphuric acid (see e.g. D. A. Armitage c.s., Inorg. Synth. *15*, 207 (1974), hydrogen chloride, phosphoric acid, and their ammonium salts (see e.g. NL—A—7613342), Lewis acids, such as boron trifluoride and aluminium trichloride (see also NL—A—7613342), bis(trialkylsilyl) sulphate (see e.g. DE—A—2649536), (fluoralkyl)sulphonic acids (see e.g. DE—A—2757936), N,O - bis(trimethylsilyl)-sulphamate (see GB—A—1,509,691) and imidazole (see e.g. D. N. Harpp c.s., J. Amer. Chem. Soc. *100*, 1222 (1978)). However, even with these catalysts high reaction temperatures, an excess of HMDS and sometimes very long reaction times, up to 48 hours, are still necessary in order to obtain a sufficient conversion to the desired silyl derivative.

It has now surprisingly been found that the silylation of many classes of organic compounds with HMDS can be accelerated considerably by using certain nitrogen containing compounds as catalysts.

The improved process of the invention relates to the trimethylsilylation of organic compounds carrying one or more active hydrogen atoms, with hexamethyldisilazane, characterized by the presence in the reaction mixture of 0.001 to 10 mol % of a catalyst of the general formula

X—NH—Y

wherein

a) X and Y are the same or different and each represents an electron-withdrawing group selected from

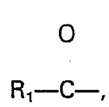, 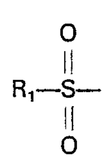 and 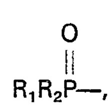,

in which $R_1$ and $R_2$ are the same or different, and each represents an alkyl group which may be substituted by one or more halogen atoms, an aryl group which may be substituted by one or more halogen atoms, alkyl, alkoxy or nitro groups, an alkoxy group, an aryloxy group which may be substituted by a halogen atom, an alkyl group or a nitro group, or a group $R_3R_4N$ in which $R_3$ and $R_4$ are the same or different and each represents a hydrogen atom, a trialkylsilyl group or an alkyl group,

b) X is an electron-withdrawing group as defined under a) and Y is a hydrogen atom or a trialkylsilyl group or,

c) X and Y together represent an electron-withdrawing group —A—Z—B— forming a cyclic system with the nitrogen atom, in which A represents a group

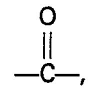

B represents a group

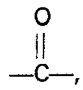 —$SO_2$—, —S—, 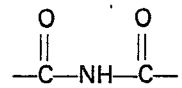

or a group

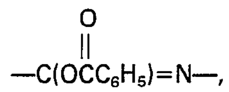

and Z represents an alkylene, alkenylene or arylene group, which groups may be substituted by one or more halogen atoms or alkyl groups.

Suitable electron-withdrawing groups in catalysts of the above formula therefore are acyl groups, sulphonyl groups and phosphoryl groups. For more details about electron-withdrawing groups see for instance: L. P. Hammet, Physical Organic Chemistry, McGraw-Hill Book Company, New York, 1970, p. 347 ff, and J. D. Roberts and M. C. Caserio, Modern Organic Chemistry, W. A. Benjamin Inc., New York, 1967, p. 553 ff.

Examples of more particularly suitable electron-withdrawing groups are represented by the formulas:

[I] 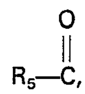

wherein $R_5$ represents an alkyl group which may be substituted by one or more halogen atoms, or an aryl group, which may be substituted by one of more alkoxy or nitro groups,

[II] 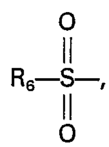

wherein $R_6$ represents a methyl group or an aryl group which may be substituted by one or more halogen atoms or methyl groups, or $R_6$ represents a group $R_7R_8N$— wherein $R_7$ and $R_8$ are the same or different and each represents a hydrogen atom, a trialkylsilyl group or an alkyl group,

[III] 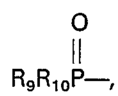

wherein $R_9$ and $R_{10}$ are the same or different and each represents an alkoxy group or an aryloxy group which may be substituted by a halogen atom or a nitro group.

Examples of particularly suitable electron-withdrawing groups which form a cyclic system together with the nitrogen atom are represented by the formulas:

[I] 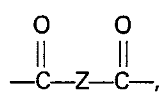

in which Z represents an alkenylene group which may be substituted by one or more halogen atoms or alkyl groups, or an arylene group which may be substituted by one or more halogen atoms,

[II] 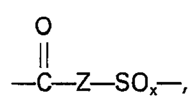

wherein x is O or 2, and Z represents an alkylene or arylene group.

Particularly preferred are those catalysts of the above general formula in which the electron-withdrawing groups are represented by the formulas:

[I] 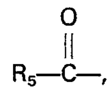

in which $R_5$ represents a dihalomethyl or trihalomethyl group, or a phenyl or naphthyl group each of which may be substituted by a methoxy group,

[II] 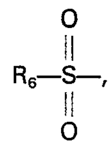

in which $R_6$ represents a methyl group, a phenyl group which may be substituted by a methyl group or a chlorine atom, an amino group, a dialkylamino group or a trialkylsilyl amino group,

[III] 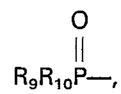

in which $R_9$ and $R_{10}$ represent a methoxy, ethoxy or propoxy group or a phenyl group which may be substituted by a nitro group or a chlorine atom.

Similarly, examples of particularly preferred electron-withdrawing groups which form a cyclic system together with the nitrogen atom are represented by the formulas:

[I] 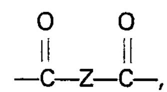

in which Z represents an ethenylene group, phenylene or naphthylene group each of which is optionally perhalo substituted,

[II] 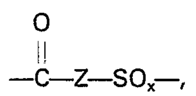

wherein x is 0 or 2, and Z represents a phenylene group.

In this description the alkyl, alkylene, alkenylene and alkoxy groups contain from one to six carbon atoms.

Examples of classes of compounds according to the above-mentioned definition possessing the desired catalytic properties are amides, sulphonamides, cyclic or open chain imides, cyclic or open chain sulphonimides, sulphamides, disulphonamides, acylphosphoramidates, sulphonylphosphoramidates and imidodiphosphates.

Suitable catalysts are for example trichloroacetamide, trifluoroacetamide, phthalimide, 3,4,5,6 - tetrachlorophthalimide, 3,4,5,6 - tetrabromophthalimide, 1,8 - naphthalimide, maleimide, barbituric acid, saccharin, N - benzoyl - 4 - toluenesulphonamide, N - (2 - methoxybenzoyl) - 4 - toluenesulphonamide, N - (1 - naphthoyl) - 4 - toluenesulphonamide, N - benzoylbenzenesulphonamide, N - (2 - methoxy - 1 - naphthoyl) - 4 - toluenesulphonamide, N - (2 - methoxy - 1 - naphthoyl)methane sulphonamide, di - (4 - toluenesulphonyl)amine, dimethyl N - (trichloroacetyl)phosphoramidate, di - 4 - nitrophenyl N - (trichloroacetyl)phosphoramidate, di - 4 - nitrophenyl N - (p - toluenesulphonyl)phosphoramidate, diisopropyl N - (dichloroacetyl)phosphoramidate, di - o - chlorophenyl N - (4 - chlorophenyl-sulphonyl)phosphoramidate, tetraphenyl imidodiphosphate, sulphamide, N,N - dimethylsulphamide, N,N' - bis(trimethylsilyl)sulphamide, 1,2 - benzisothiazol - 3 - (2H) - one and 4 - benzoyloxy - 1,2 - dihydro - 1 - oxo - phthalazine.

Particularly preferred catalysts are saccharin, di - 4 - nitrophenyl N - (trichloroacetyl)-phosphoramidate, di - 4 - nitrophenyl N - (4 - toluenesulphonyl)phosphoramidate and tetraphenyl imidodiphosphate.

The reaction may be carried out with or without an organic solvent at temperatures in the range of 0°C to 150°C. The solvent, if any, must be inert to the reactants as well as to the products and preferably will dissolve little or nothing of the ammonia generated in the reaction at the temperature at which the reaction is carried out, because due to equilibrium institution the rate of reaction will slow down at higher ammonia concentrations. Suitable solvents are straight, branched or cyclic hydrocarbons, which may be substituted

by one or more halogen atoms, for example hexane, cyclohexane, dichloromethane and chloroform, aromatic hydrocarbons, for example benzene, toluene and xylene, alkyl esters of carboxylic acids, for example ethyl acetate and butyl acetate, nitriles, for example acetonitrile and benzonitrile, dimethylformamide, dimethylsulphoxide, or mixtures thereof.

Organic compounds carrying one or more —OH, —NH, —$NH_2$ or —SH groups can be silylated by the method of the present invention. Examples of such compounds are alcohols, amines, phenols, thiophenols, acids, amides, sulphonamides, thioamides, phosphoramides, aminoacids, heterocyclic compounds, penicillanic and cephalosporanic acids derivatives, hydrazines, N-hydroxy-succinimides, hydroxylamines, thiols and enolisable ketones. Due to the large number of classes of organic compound which have one or more —OH, —NH, —$NH_2$ or —SH groups, the above enumeration is not to be considered to be limiting upon the general scope of the present invention.

By using the catalysts of the present invention the original disadvantages of the use of HMDS as a silylating agent, i.e. long reaction times and/or high reaction temperatures and/or the use of a large excess of the silylating agent have been overcome.

Now, the silylation reactions can be performed in a short time and/or at low reaction temperatures and a small excess of silylating agent is usually sufficient. Furthermore, at these reaction conditions a cleaner reaction mixture is formed, through which a purer product and in many cases a higher yield is obtained. Another advantage of the improved method of the present invention is that it has now become possible to silylate compounds of which it is known that they do not react with HMDS, in a short time by using the catalysts of the invention. Examples of such compounds are tertiary alcohols (see S. H. Langer c.s. J. Org. Chem. *23*, 50 (1958)) and phthalimide (see D. N. Harpp c.s., J. Amer. Chem. Soc. *100*, 1222 (1978)).

Furthermore, by applying the process of the invention the preparation of N,O - bis(trimethylsilyl) derivatives of penicillanic acnd cephalosporanic acid derivatives can be carried out in a simple way and with quantitative yields. These derivatives can be prepared in another way only with difficulty. (see e.g. F. Bortesi c.s., J. Pharm. Sci. *66*, 1767 (1977)).

Another advantage of the present process for the preparation of silylated compounds exists in the fact that, as far as these compounds in their turn are used as silylating agents, such as for instance N - trimethylsilylimidazole, N,N' - bis(trimethylsilyl)urea etc., these compounds are not contaminated with ammonium salts, which salts may lead to undesired side-reactions when using these silylating agents.

An example which discloses the present invention is the silylation of urea. Silylation of urea without a catalyst takes 36 hours at circa 125°C, as described in NL—A—7613342. Using ammonium chloride as a catalyst it still takes 6 hours at 118°C (Example III of the forementioned patent). However, in the present invention, using saccharin as a catalyst, it has been found that a reaction time of 20 minutes suffices for completion of the reaction. Another example is the reaction of phenylhydrazine with HMDS. Without catalyst a yield of 12% was obtained after 12 hours at 130°C (R. Fessenden c.s. J. Org. Chem. *26*, 4638 (1961)). Using ammonium chloride as a catalyst, a yield of 89% was obtained using the same reaction conditions. Using saccharin as a catalyst, only 2.5 hours were required for obtaining the same yield of silylated product.

Still another example is the conversion of 5,5 - dimethylhydantoin into its N,N' - bis(trimethylsilyl) derivative. According to example 2 of GB 1,509,691 a yield of 86% of crude product was reached in 7.25 hours in refluxing toluene while using a more than 100% excess of hexamethyldisilazane and 1 mol% of N,O - bis(trimethylsilyl)sulphamate as the catalyst. Under the same conditions 0.07 mol% of saccharin effected an actual yield of 88.3% in 1.5 hours, while employing only 27% excess of hexamethyldisilazane.

Reactions of HMDS with tertiary alcohols, which were known do not react with HMDS, even in the presence of trimethylchlorosilane as a catalyst (see S. H. Langer c.s. J. Org. Chem. *23*, 50 (1958)), now proceed fast to very fast under the influence of the above-described catalysts. For example, t-amyl alcohol reacts in three hours with HMDS to form the trimethylsilyl ether using saccharin as a catalyst, and 2-methyl-2-hexanol reacts in only 15 minutes to form the trimethylsilyl derivative in a yield of 92% using di - 4 - nitrophenyl N - (4 - toluenesulphonyl)phosphoramidate as a catalyst. The reaction of phthalimide with HMDS also shows the advantages of the use of the catalysts of the invention. Silylation of phthalimide with hexamethyldisilazane with imidazole as a catalyst requires a two days' reflux (D. N. Harpp c.s., J. Amer. Chem. Soc. *100*, 1222 (1978)). It has now been found that the saccharin catalyzed silylation of phthalimide with HMDS goes to completion within 1.5 hours and gives substantially higher yields.

Generally thiols seem to constitute a class of difficult substrates, the trimethylsilylation of which often requires the use of enforced conditions and considerable excess of silylation agent, when not employing a combination of e.g. trimethylchlorosilane with triethylamine. Therefore, as published in the Journal of Organometallic Chemistry, *25*, pp. 389—393 (1970), Bassindale and Walton seem to prefer a more sophisticated method for the conversion of arene thiols, involving preliminary preparation of lithium arenethiolates by the action of e.g. phenyllithium, followed by reaction with trimethyl chlorosilane.

Not easy trimethylsilylation of certain compounds containing a silylatable thio group is also apparent from DE 1 959 523, wherein 5-substituted thiouracils are converted into 2 - trimethylsilylthio - 4 - trimethylsilyloxy - 5 - substituted - pyrimidines. In spite of the condition prevailing, that the trimethylsilyl groups introduced replace mobile hydrogen atoms under simultaneous generation of endocyclic double bonds, so that the formation of the trimethylsilylthio group may not be so difficult as compared with the trimethylsilylation of various thiols not subject to tautomerisation, the conditions nevertheless involve at

least two hours boiling of a solution of 10 g of a thiouracil in a very large excess of hexamethyldisilazane, i.e. 100 ml, while employing a relatively large amount of catalyst, i.e. 2 ml of trimethyl chlorosilane.

That thiols are difficult substrates for the trimethylsilylation by means of hexamethyldisilazane and catalyst also emerges from an article by R. S. Glass in the Journal of Organometallic Chemistry, *61*, pp. 83—90 (1973), but the catalyst employed therein, i.e. imidazole, which in view of the accompanying discussion may also act by way of its trimethylsilyl derivative continually regenerated in situ, and which may have a special disposition towards silylation of thiols, is indeed able to effect a practically useful trimethylsilylation of primary alkane thiols, of activated thiols like benzhydryl thiols and of arene thiols like benzene thiol.

As it has been found now, at least the more powerful catalysts of the present process, e.g. saccharin, are at least as effective as imidazole in the trimethylsilylation of generally arene thiols by hexamethyldisilazane, and can be used also for an efficient preparation of novel trimethylsilylthio derivatives of certain heterocyclic compounds.

Accordingly, another feature of the invention is the preparation of trimethylsilyl substituted compounds of the general formula

$$R—S—SiMe_3$$

in which formula R represents a five- or six-membered heterocyclic group having one or more nitrogen or sulphur atoms as the hetero atoms, which group may be substituted by one or more alkyl groups, a phenyl group, a trimethylsilyl group attached to a ring nitrogen atom, an alkylamino group or a trimethylsilyloxycarbonylmethyl group.

Examples of the above-mentioned group R are a 1,3,4 - thiadiazolyl group, a 1,2,3,4 - tetrazolyl group, a 1,2,3 - triazolyl group, a 1,2,4 - triazolyl group, an imidazolyl group or a pyrimidyl group, which may be substituted by a methyl group, a phenyl group, a methylamino group, a trimethylsilyl group attached to a ring nitrogen atom or a trimethylsilyloxycarbonylmethyl group. Compounds according to the above formula are for example 2 - trimethylsilylthio - 5 - methyl - 1,3,4 - thiadiazole, 1 - methyl - 5 - trimethylsilylthio - 1H - tetrazole, 1 - trimethylsilyl - 5 - trimethylsilylthio - 1,2,3 - 1H - triazole, 1 - methyl - 2 - (trimethylsilylthio)imidazole, 1 - trimethylsilyl - 3 - trimethylsilylthio - 1H - 1,2,4 - triazole, 1 - phenyl - 5 - trimethylsilylthio - 1H - tetrazole, 4,6 - dimethyl - 2 - (trimethylsilylthio)pyrimidine, 2 - methylamino - 5 - trimethylsilylthio - 1,3,4 - thiadiazole, trimethylsilyl 5 - trimethylsilylthio - 1H - tetrazolyl - 1 - acetate, trimethylsilyl 5 - trimethylsilylthio - 1,3,4 - thiadiazolyl - 2 - acetate. These trimethylsilylated thiols are useful intermediates in the preparation of valuable compounds by a new method.

Example of such valuable compounds are therapeutically active 3′ - thio - substituted cephalosporins or intermediates therefore. They are prepared by reacting the trimethylsilylated thiols with the corrresponding 3′ - halo substituted cephalosporins. This new method for the preparation of these cephalosporins is described in the European patent application No. 0047560. Other applications of trimethylsilylthio compounds have been described for instance by Mukaiyama c.s. (Chem. Lett., 187, 1974 and Chem. Lett., 1013, *1974*) and by Evans c.s. (J. Amer. Chem. Soc., *99*, 5009 (1977)).

Still another feature of the invention is the preparation of the group of new compounds comprising trimethylsilyl 7 - phenylacetamido - 3 - (1 - methyl - 1H - tetrazolyl - 1 - thio)methyl - 3 - cephem - 4 - carboxylate 1 - oxide, N,O - bis(trimethylsilyl) - 7 - phenylacetamido - 3 - (1H - 1,2,3 - triazolyl - 5 - thio)methyl - 3 - cephem - 4 - carboxylic acid 1 - oxide, trimethylsilyl 7 - trimethylsilylamino - 3 - (5 - methyl - 1,3,4 - thiadiazolyl - 2 - thio)methyl - 3 - cephem - 4 - carboxylate, trimethylsilyl 7 - trimethylsilylamino - 3 - (1 - trimethylsilyl - 1H - 1,2,3 - triazolyl - 5 - thio)methyl - 3 - cephem - 4 - carboxylate, trimethylsilyl 7 - trimethylsilylamino - 3 - (1 - methyl - 1H - tetrazolyl - 5 - thio)methyl - 3 - cephem - 4 - carboxylate, trimethylsilyl 7 - phenylacetamido - 3 - (1 - methyl - 1H - tetrazolyl - 5 - thio)methyl - 3 - cephem - 4 - carboxylate and trimethylsilyl 7 - phenylacetamido - 3 - (1 - trimethylsilyl - 1H - 1,2,3 - triazolyl - 5 - thio)methyl - 3 - cephem - 4 - carboxylate. These compounds are valuable compounds for the preparation of therapeutically active cephalosporins.

It will be clear for a person skilled in the art that the catalyst may be added to the reaction mixture as such, but also by way of a derivative thereof, and preferably in the form of a silylated derivative, which decomposes in the reaction mixture to the above mentioned catalytic compounds.

The following examples are provided to further illustrate the process of this invention, however without limitation of the invention to the application to these compounds.

Example 1

Preparation of 1-trimethylsilyloxydodecane

A. Saccharin (90 mg; 0.5 mmole) was added to 18.6 g (0.1 mole) of 1-dodecanol and the mixture was heated to 130°C. Hexamethyldisilazane (15.6 ml; 0.075 mole) was added during 8 minutes. The ammonia produced in the reaction was passed by means of a stream of dry nitrogen into water and tritrated with 1N HCl. It was found that the calculated amount of ammonia was evolved during 15 minutes after the beginning of the addition of the hexamethyldisilazane. Refluxing was continued for 10 minutes, the excess

of hexamethyldisilazane was distilled off at reduced pressure and the residue was vacuum distilled to yield 25.42 g (98.5%) of 1 - trimethylsiloxydodecane, b.p. 120°C/0.67 mbar (0.5 mm Hg); $N_D^{25}$ 1.4268.

B. Hexamethyldisilazane (7.8 ml; 38 mmoles) was added to a refluxing solution of 9.3 g (50 mmoles) of 1-dodecanol and 51 mg (0.27 mmole) of saccharin in 50 ml of dichloromethane. By the method described in Example 1A it was found that the calculated amount of ammonia was evolved after refluxing for one hour.

C. A solution of 9.30 g (50 mmoles) of 1-dodecanol and 70 mg (0.25 mmole) of dimethyl N - trichloroacetylphosphoramidate in 50 ml of dichloromethane was heated to reflux and hexamethyldisilazane (7.8 ml; 37.5 mmoles) was rapidly dropped in by means of a pressure equalized dropping funnel. It was found that the calculated amount of ammonia was evolved after refluxing for 75 minutes.

D. This preparation was carried out as described in Example 1C, but 121 mg (0.25 mmole) of di - 4 - nitrophenyl N - trichloroacetylphosphoramidate was used as the catalyst.

The reaction ran to completion in 40 minutes.

E. Using 121 mg (0.25 mmole) of di - 4 - nitrophenyl N - 4 - toluenesulphonylphosphoramidate as the catalyst, the reaction time was 60 minutes.

Example 2

Preparation of trimethylsilyloxycyclohexane

By the method described in Example 1A cyclohexanol (15.0 g; 0.15 mole) was silylated with 23.4 ml (0.112 mole) of hexamethyldisilazane, which were added during 5 minutes. The silylation was catalysed with 137 mg (0.75 mmole) of saccharin. The calculated amount of ammonia was evolved in 18 minutes. Refluxing was then continued for 10 minutes, the excess of hexamethyldisilazane was distilled off at reduced pressure and the residue was vacuum distilled to yield 22.01 g (85.3%) of trimethylsilyloxycyclohexane, b.p. 53—55°C/16 mbar (12 mm Hg), $n_D^{25}$ 1.4281.

Example 3

Preparation of 2-trimethylsilyloxy-2-methylbutane

Hexamethyldisilazane (21.9 ml; 0.105 mole) was added to a refluxing mixture consisting of 17.6 g (0.20 mole) of 2 - methyl - 2 - butanol (t - amyl alcohol) and 0.18 g (1 mmole) of saccharin. The course of the silylation was followed as described in Example 1A. It was found that 50% of the calculated amount of ammonia was evolved in 18 minutes. Refluxing was continued for 3.25 hours, after which time 98% of the calculated amount of ammonia was evolved. Distillation at normal pressure yielded 22.75 g (71%) of pure 2 - trimethylsilyloxy - 2 - methylbutane, b.p. 129—130°C; $n_D^{22}$ 1.3980. A forerun with b.p. 125—129°C (3.69 g) with $N_D^{22}$ 1.3974 contained 88% of the title compound according to NMR analysis.

Example 4

Preparation of 17-beta-trimethylsilyloxy-4-androsten-3-one

Hexamethyldisilazane (246 mg; 1.5 mmole) was added to a refluxing suspension of 577 mg (2.0 mmoles) of 17 - beta - hydroxy - 4 - androsten - 3 - one and 1.8 mg (0.01 mmole) of saccharin in 10 ml of dichloromethane. The course of the reaction was followed by means of thin layer chromatography on Kieselgel 60 $F_{254}$ (Merck) with a 9+1 mixture of toluene and acetone as the eluens. It was found that after refluxing for 2 hours the starting material was no longer present and that one new product had been formed. By means of NMR spectroscopy it was established that the product, obtained in quantitative yield after evaporation of the solvent in vacuo, was pure 17- beta- trimethylsilyloxy - 4 - androsten - 3 - one.

The melting point of the product was 126—128°C (dec.).

Example 5

Preparation of 1-trimethylsilyloxy-2-propene

Allyl alcohol (24.28 g; 0.418 mole) and saccharin (0.36 g; 2 mmoles) were added to 50 ml of pentane and the mixture was heated to the reflux temperature. Hexamethyldisilazane (51 ml; 0.25 mole) was added to the mixture during 7 minutes. By titration of the ammonia generated during the reaction it was found that the calculated amount of it was evolved in 1.5 hours. Distillation at normal pressure yielded 46.1 g (85%) of 1 - trimethylsilyloxy - 2 - propene, b.p. 97—100°C, $n_D^{25}$ 1.3943.

Example 6

Preparation of penta(trimethylsilyl)fructose

Hexamethyldisilazane (42 ml; 0.20 mole) was added dropwise in 8 minutes to a refluxing mixture of 7.20 g (40 mmoles) of fructose, 0.07 g (0.4 mmole) of saccharin, 24 ml of chloroform and 8 ml of pyridine. The ammonia evolved was absorbed in water and titrated with 1N $H_2SO_4$. It was found that 0.10 mole of ammonia was evolved after refluxing for one hour. Refluxing was continued for half an hour, the solvents were distilled off at normal pressure and the residue was vacuum distilled to yield 19.63 g (90.9%) of penta(trimethylsilyl)fructose, b.p. 138—142°C/0.67 mbar (0.5 mm Hg); $n_D^{25}$ 1.4306.

Example 7

Preparation of 1-trimethylsilyloxyhexane

A. 5.10 g (50 mmoles) of 1-hexanol were mixed with 0.37 g (2.5 mmoles) of phthalimide and heated in an oil bath to 130°C. Hexamethyldisilazane (7.8 ml; 37.5 mmoles) was added and the evolution of ammonia

was followed by absorbing it in water and titrating it with 1N $H_2SO_4$. It was found that the calculated amount of 25 mmoles of ammonia was evolved in 130 minutes.

B. The experiment was repeated with 2.5 mmoles of 3,4,5,6 - tetrachlorophthalimide as the catalyst. The calculated amount of ammonia was evolved in 70 minutes.

C. The experiment was also repeated with 2.5 mmoles of 3,4,5,6 - tetrabromophthalimide as the catalyst. It was found that the calculated amount of ammonia was evolved in 20 minutes.

D. A run without the addition of catalyst was also carried out and in that case it was found that the calculated amount of ammonia was evolved after refluxing for 205 minutes.

E. 5.10 g (50 mmoles) of 1-hexanol were mixed with the catalysts mentioned in the following table, heated in an oil bath of 130°C and 7.8 ml (37.5 mmoles) of hexamethyldisilazane were added. The time (t) in which half the theoretical amount of ammonia was evolved was measured. Further details are to be found in the following table.

| Catalyst | Mol% of catalyst | t (minutes) |
|---|---|---|
| none | — | 22 |
| succinimide | 5.0 | 18 |
| 3,3-dimethylglutarimide | 5.0 | 16 |
| maleimide | 5.0 | 9 |
| 1,8-naphthalimide | 5.0 | 8 |
| 1,2-benzisothiazol-3(2H)-one | 5.0 | 9 |
| 4-benzoyloxy-1,2-dihydro-1-oxo-phthalazine | 5.0 | 7 |
| 3,4,5,6-tetrabromophthalimide | 2.0 | 4 |
| 3,4,5,6-tetrachlorophthalimide | 2.0 | 4 |
| barbituric acid | 2.0 | 12 |
| dimethyl N-trichloroacetylphosphoramidate | 0.1 | 7 |
| saccharin | 0.5 | 4 |
| di-4-nitrophenyl N-trichloroacetylphosphoramidate | 0.1 | 1.5 |
| di-4-nitrophenyl N-4-toluenesulphonylphosphoramidate | 0.1 | 1.5 |
| di-4-nitrophenyl N-trichloroacetylphosphoramidate | 0.01 | 3 |
| di-4-nitrophenyl N-4-toluenesulphonylphosphoramidate | 0.001 | 6 |
| tetraphenyl imidodiphosphate | 0.1 | 1 |
| tetraphenyl imidodiphosphate | 0.001 | 13 |

Example 8

Preparation of (2-methyl-2-hexyloxy)trimethylsilane

A mixture of 5.80 g (50 mmoles) of 2-methyl-2-hexanol and 25 mg (0.05 mmole) of di - 4 - nitrophenyl N - 4 - toluenesulphonylphosphoramidate was placed in an oil bath of 140°C and 7.8 ml (37.5 mmoles) of hexamethyldisilazane were added. It was found that the calculated amount of ammonia was evolved after refluxing for 15 minutes. Vacuum distillation yielded 8.66 g (92%) of (2 - methyl - 2 - hexyloxy)-trimethylsilane; b.p. 54—60°C/23.9 mbar (18 mm Hg); $n_D^{25}$ 1.4074.

Example 9

Reaction of hexamethyldisilazane with water

To a refluxing solution of 740 mg (41 mmoles) of water and 21.4 mg (0.12 mmole) of saccharin in 15 ml of dry acetonitrile, 12.5 ml (60 mmoles) of hexamethyldisilazane were added dropwise within 5 minutes. The ammonia evolved was led into water by means of a stream nitrogen and titrated with 1N sulphuric acid. Already after 4 minutes from the start of the dropwise addition, 50% of the calculated amount of

ammonia had been collected. After 35 minutes no more ammonia came free and the calculated amount had been collected.

Example 10

Preparation of N-trimethylsilyl-p-toluidine

Hexamethyldisilazane (25 ml; 0.12 mole) was added in 5 minutes to a mixture of 17.25 g (0.16 mole) of p-toluidine and 0.15 g (0.8 mmole) of saccharin, which was heated to 130°C in an oil bath. By titrating the ammonia evolved during the reaction it was found that the calculated amount of it was evolved after refluxing for 2 hours. Refluxing was continued for half an hour and the reaction mixture was vacuum distilled to yield 24.0 g (83%) of N - trimethylsilyl - p - toluidine, b.p. 98—102°C/16—17.3 mbar (12—13 mm Hg).

Example 11

Preparation of phenoxy(trimethyl)silane

To a solution of 19 g (0.2 mole) of phenol and 80 mg (0.4 mmole) of saccharin in 15 ml of dichloromethane boiling under reflux, hexamethyldisilazane (31 ml; 0.15 mmole) was added dropwise. After 25 minutes the calculated amount of ammonia had been evolved as could be established by titration in the manner described herebefore. Fractionation yielded 31.6 g (95%) of phenoxy - (trimethyl)silane; b.p. 62—63°C/16 mbar (12 mm Hg); $n_D^{26}$ 1.4731.

Example 12

Preparation of o-trimethylsilyloxytoluene

10.80 g (0.1 mole) of o-cresol were dissolved in 30 ml of dichloromethane. Saccharin (90 mg; 0.5 mmole) was added, the mixture heated to reflux and hexamethyldisilazane (15.6 ml; 0.075 mole) was added. It was found that the theoretical amount of ammonia was evolved in 30 minutes. After evaporation of the solvent and the excess of hexamethyldisilazane the residue was vacuum distilled to yield 16.91 g (93.9%) of o-trimethylsilyloxytoluene, b.p. 46—53°C/0.67—0.93 mbar (0.5—0.7 mm Hg); $n_D^{25}$ 1.4756.

A parallel run without saccharin needed 3.75 hours of refluxing to evolve the calculated amount of ammonia.

Example 13

Preparation of (2,6-di-sec-butylphenoxy) trimethylsilane

Hexamethyldisilazane (7.8 ml; 37.5 mmoles) was added to a refluxing mixture of 10.4 g (50 mmoles) of 2,6 - di - sec - butylphenol, 23 mg (0.05 mmole) of di - 4 - nitrophenyl N - trichloroacetylphosphoramidate and 20 ml of chloroform. The evolution of ammonia stopped after refluxing for 3 hours. The chloroform was distilled off at reduced pressure and the residue was fractionated to yield 12.64 g (90%) of the title compound, b.p. 86—90°C/0.53 mbar (0.4 mm Hg); $n_D^{25}$ 1.4812.

Example 14

Preparation of trimethyl (phenylthio)silane

A. Hexamethyldisilazane (23.4 ml; 0.11 mole) was added during 10 minutes to a refluxing solution of 16.0 g (0.145 mole) of thiophenol and 135 mg (0.75 mmole) of saccharin in 25 ml of chloroform. By titration of the ammonia produced during the reaction it was found that the silylation was completed after refluxing for 2.66 hours. The solvent and the excess of hexamethyldisilazane were distilled off at reduced pressure and the residue was fractionated to yield 24.4 g (92.1%) of trimethyl(phenyl-thio)silane, b.p. 92—95°C/16—17.3 mbar (12—13 mm Hg), $n_D^{25}$ 1.5270.

B. A mixture of 17.4 g (0.158 mole) of thiophenol, 135 mg (0.75 mmole) of saccharin and 24.7 ml (0.12 mole) of hexamethyldisilazane was refluxed for 2 hours. Distillation yielded 23.9 g (83%) of trimethyl(phenylthio)silane, b.p. 88—90°C/16 mbar (12 mm Hg), $n_D^{25}$ 1.5308.

Example 15

Preparation of 2-trimethylsilylthio-5-methyl-1,3,4-thiadiazole

Hexamethyldisilazane (15.6 ml; 0.075 mole) was added to a refluxing solution of 13.2 g (0.1 mole) of 2 - mercapto - 5 - methyl - 1,3,4 - thiadiazole and 92 mg (0.5 mmole) of saccharin in 25 ml of toluene. By titrating the ammonia evolved it was found that the reaction was completed after refluxing for 30 minutes. The toluene was distilled off at normal pressure and the residue was vacuum distilled. There were obtained 18.63 g (91.3%) of 2 - trimethylsilylthio - 5 - methyl - 1,3,4 - thiadiazole, b.p. 150—152°C/20 mbar (15 mm Hg). The distillate turned into a solid with m.p. 67—69°C. NMR (60 MHz; in $CCl_4$ with tetramethylsilane (delta=0) as internal standard): two singlets at 0.56 and 2.42, integration ratio 3:1.

Example 16

Preparation of 1-methyl-5-trimethylsilylthio-1H-tetrazole

0.582 g (5.0 mmoles) of 5 - mercapto - 1 - methyl - 1H - tetrazole and 5.0 mg (0.03 mmole) of saccharin were dissolved in a mixture of 12 ml of ethyl acetate and 25 ml of dichloromethane. The mixture was refluxed and hexamethyldisilazane (1.26 ml; 5.5 mmoles) was added. The evolution of ammonia

stopped after refluxing for one hour. Volatile material was evaporated in vacuo to yield 0.94 g of 1 - methyl - 5 - trimethylsilylthio - 1H - tetrazole. NMR spectrum ($CCl_4$): 2 singlets at 0.61 and 3.79 ppm, integration ratio 3:1.

Example 17

Preparation of 1-trimethylsilyl-5-trimethylsilylthio-1,2,3-1H-triazole

Hexamethyldisilazane (1.52 ml; 7.3 mmoles) was added to a refluxing mixture of 0.49 g (4.86 mmoles) of 5 - mercapto - 1,2,3 - (1H)triazole, 5 mg (0.027 mmole) of saccharin, 10 ml of ethyl acetate and 15 ml of dichloromethane. The ammonia evolved was titrated with 1N $H_2SO_4$ by the method described in Example 1A and it was found that 2 equivalents (4.9 mmoles) of it were evolved after refluxing for 30 minutes. Volatile materials were evaporated in vacuo and from the residue (1.19 g; 96%) a NMR spectrum was taken in carbon tetrachloride solution: delta 0.31 (9H); 0.48 (9H); 7.46 (1H).

Example 18

Preparation of 1-methyl-2-(trimethylsilylthio)imidazole

To a mixture consisting of 1.14 g (10 mmoles) of 1 - methyl - 2 - mercaptoimidazole, 18 mg (0.1 mmole) of saccharin and 20 ml of toluene, which mixture was boiling under reflux while a stream of nitrogen was passed over, hexamethyldisilazane (1.5 ml; 7.2 mmoles) was added.

The ammonia evolved was led into water by means of the nitrogen stream. By titration with 1N sulphuric acid it was established that the calculated amount of ammonia had been evolved within 40 minutes. The mixture was evaporated to dryness under reduced pressure and the remaining residue was dried at room temperature under vacuum, yielding 1.78 g (95%) of 1 - methyl - 2 - (trimethylsilylthio)imidazole, m.p. 49—52°C.

$^1H$ NMR ($CCl_4$): 0.55 (s, 9H); 3.49 (s, 3H); 6.48 (d, 1H, J 2 Hz); 6.69 (d, 1H, J 2 Hz).

Example 19

Preparation of 1-trimethylsilyl-3-trimethylsilylthio-1H-1,2,4-triazole

To a suspension of 9.70 g (96 mmoles) of 3 - mercapto - 1H - 1,2,4 - triazole and 100 mg (0.25 mmole) of di - 4 - nitrophenyl N - (4 - toluenesulphonyl)phosphoramidate in 200 ml of dichloromethane, which suspension was boiling under reflux while a stream of dry nitrogen was passed over, 29.2 ml (0.14 mole) of hexamethyldisilazane were added dropwise quickly. The ammonia evolved was led into water by means of the nitrogen stream. By titration with 1N sulphuric acid it was established that the calculated amount of ammonia (96 mmoles) had been evolved within 1.25 hours. Boiling was continued for another 0.5 hour and then the clear colourless solution was evaporated to dryness at a rotating film evaporator, yielding 23.1 g (98%) of 1 - trimethylsilyl - 3 - trimethylsilylthio - 1H - 1,2,4 - triazole; m.p. 90—94°C.

$^1H$ NMR ($CCl_4$): 0.52 (s, 9H); 0.55 (s, 9H); 7.52 (s, 1H).

Example 20

Preparation of 1-phenyl-5-trimethylsilylthio-1H-tetrazole

In the same manner as described in Example 19 1.78 g (10 mmoles) of 1 - phenyl - 5 - mercapto - 1H - tetrazole in 50 ml of 1,2 dichloroethane was silylated with 2.60 ml (12.4 mmoles) of hexamethyldisilazane with 5 mg (0.03 mmole) of saccharin as the catalyst. The calculated amount of ammonia was collected within 20 minutes. Boiling was continued for another 10 minutes and then the mixture was evaporated to dryness and the residue was dried, yielding 2.58 g (103%) of 1 - phenyl - 5 - trimethylsilylthio - 1H - tetrazole; m.p. 67—68°C.

$^1H$ NMR ($CCl_4$): 0.68 (s, 9H); 7.38—7.64 (m, 3H); 7.91—8.17 (m, 2H).

Example 21

Preparation of trimethylsilyl(4-chlorophenylthio)silane

A mixture of 14.50 g (0.10 mole) of 4-chlorothiophenol and 45 mg (0.25 mmole) of saccharin in a Claisen vessel was heated in an oil bath up to 120°C, while passing a stream of dry nitrogen over the mixture. Hexamethyldisilazane (20.8 ml, 0.10 mole) was added dropwise quickly to the mixture. Thereby a precipitate was formed that disappeared again after heating for 10 minutes. The progress of the reaction was supervised by leading the ammonia evolved by means of the nitrogen stream into water and titrating with 1N sulphuric acid. It was established that the calculated amount of ammonia had been evolved within 40 minutes. The product was isolated by fractionation under reduced pressure, yielding 19.81 g (91.5%) of trimethylsilyl(4 - chlorophenylthio)silane; b.p. 82—84°C/2.66 mbar (2.0 mm Hg).

Example 22

Preparation of trimethyl(4-methylphenylthio)silane

A. According to the procedure described in Example 21 14.70 g (118 mmoles) of 4 - methylthiophenol were silylated with 25 ml (120 mmoles) of hexamethyldisilazane using 40 mg (0.08 mmole) of tetraphenyl imidodiphosphate as the catalyst. It was established that the silylation was completed within 1 hour. The product was isolated by distillation under vacuum, yielding 22.16 g (95.4%) of trimethyl(4 - methylphenylthio)silane; b.p. 80.5—82.0°C/3.3 mbar (2.5 mm Hg).

B. In a second run 11.35 g (91.5 mmoles) of 4 - methylthiophenol and 20.8 ml (100 mmoles) of hexamethyldisilazane were used, while applying 200 mg (0.42 mmole) of tetraphenyl imidodiphosphate. With this amount of the catalyst it was found that the reaction was already fully completed within 30 minutes. By distillation trimethyl (4 - methylphenylthio)silane was isolated in a yield of 84%; b.p. 79—81°C/3.3 mbar (2.5 mm Hg).

Example 23

Preparation of 4,6-dimethyl-2-(trimethylsilylthio)pyrimidine

To a mixture consisting of 0.70 g (5.0 mmoles) of 4,6 - dimethyl - 2 - mercaptopyrimidine, 10 mg (0.02 mmole) of tetraphenyl imidodiphosphate and 25 ml of toluene, which mixture was boiling under reflux, 1.0 ml (4.8 mmoles) of hexamethyldisilazane was added. According to the procedure described in Example 19 it was established that the calculated amount of ammonia had been evolved after boiling for 1.5 hours. The mixture was then evaporated to dryness and the residue was dried, yielding 1.08 g (102%) of 4,6 - dimethyl - 2 - (trimethylsilylthio)pyrimidine).

$^1H$ NMR ($CCl_4$): 0.43 (s, 9H); 2.32 (s, 6H); 6.63 (s, 1H).

Example 24

Preparation of 2-methylamino-5-trimethylsilylthio-1,3,4-thiadiazole

To a solution of 298 mg (2.03 mmoles) of 2 - methylamino - 5 - mercapto - 1,3,4 - thiadiazole and 2 mg (0.004 mmole) of tetraphenyl imidodiphosphate in 10 ml of ethyl acetate, which solution was boiling under reflux, 0.42 ml (2.0 mmoles) of hexamethyldisilazane was added. According to the procedure described in Example 19 it was established that the evolution of ammonia came to an end after boiling for 0.5 hour. At that time 1 mmole of ammonia had been collected. Boiling was continued for another 0.5 hour and then the solvent and other volatile materials were removed at a rotating film evaporator, yielding 434 mg (97%) of 2 - methylamino - 5 - trimethylsilylthio - 1,3,4 - thiadiazole), m.p. 80—82°C.

$^1H$ NMR ($CCl_4$): 0.60 (s, 9H); 2.90 (d, 3H, J 5.5 Hz); 5.85 (q, 1H, J 5.5 Hz).

Example 25

Preparation of trimethyl(4-bromophenylthio)silane

According to the procedure described in Example 21 10.82 g (57.2 mmoles) of 4 - bromothiophenol were silylated with 8.9 ml (42.6 mmoles) of hexamethyldisilazane, using 25 mg (0.05 mmole) of di - 4 - nitrophenyl N - (4 - toluenesulphonyl)phosphoramidate as the catalyst. The calculated amount of ammonia had been collected after boiling under reflux during 15 minutes. Fractionation under reduced pressure yielded 13.16 g (88.4%) of trimethyl (4 - bromophenylthio)silane, b.p. 87—88°C/1.06 mbar (0.8 mm Hg), $n_D^{25}$ 1.5652.

Example 26

Preparation of trimethyl(3,4-dichlorophenylthio)silane

According to the procedure described in Example 21 8.45 g (47.2 mmoles) of 3,4 - dichlorothiophenol were silylated with 7.40 ml (35.4 mmoles) of hexamethyldisilazane, using 22 mg (0.045 mmole) of tetraphenylimidodiphosphate as the catalyst. The calculated amount of ammonia had been evolved within 35 minutes. Fractionation under reduced pressure yielded 11.28 g (95.3%) of trimethyl(3,4 - dichlorophenylthio)silane, b.p. 96—97°C/1.06 mbar (0.8 mm Hg), $n_D^{25}$ 1.5600.

Example 27

Preparation of trimethylsilyl 5-trimethylsilylthio-1H-tetrazolyl-1-acetate

To a mixture consisting of 318 mg (2.0 mmoles) of 5 - mercapto - 1H - tetrazolyl - 1 - acetic acid, 5.5 mg (0.03 mmole) of saccharin and 25 ml of toluene, which mixture was boiling under reflux, hexamethyldisilazane (0.6 ml; 2.9 mmoles) was added. After boiling for 2 hours the evolution of ammonia came to an end. At that time 2 mmoles of ammonia had been collected. The mixture was evaporated to dryness under reduced pressure and the residue was dried, yielding 0.60 g (100%) of trimethylsilyl 5 - trimethylsilylthio - 1H - tetrazolyl - 1 - acetate as a viscous oil.

$^1H$ NMR ($CCl_4$): 0.29 (s, 9H); 0.65 (s, 9H); 4.87 (s, 2H).

Example 28

Preparation of trimethylsilyl 5-trimethylsilylthio-1,3,4-thiadiazolyl-2-acetate

To a mixture consisting of 0.44 g (2.5 mmoles) of 5-mercapto 1,3,4 - thiadiazolyl - 2 - acetic acid, 5.0 mg (0.025 mmole) of saccharin and 30 ml of dichloromethane, which mixture was boiling under reflux, 0.9 ml (4.3 mmoles) of hexamethyldisilazane was added. The calculated amount of ammonia (2.5 mmoles) had been evolved after boiling for 1.5 hours. The mixture was then evaporated to dryness and the residue was dried under reduced pressure, yielding 0.80 g (100%) of trimethylsilyl 5 - trimethylsilylthio - 1,3,4 - thiadiazolyl - 2 - acetate, m.p. 45—49°C.

$^1H$ NMR ($CCl_4$): 0.33 (s, 9H); 0.60 (s, 9H); 3.73 (s, 2H).

Example 29

Preparation of trimethylsilyl benzoate

A. Hexamethyldisilazane (15.6 ml; 0.075 mole) was added during 5 minutes to a refluxing solution of 12.2 g (0.1 mole) of benzoic acid and 90 mg (0.5 mmole) of saccharin in 30 ml of dichloromethane. By titration of the ammonia evolved it was found that the calculated amount of it was produced in 40 minutes after starting the addition of the hexamethyldisilazane. The solvent was distilled off at normal pressure and the residue was vacuum distilled to yield 17.80 g (91.7%) of trimethylsilyl benzoate, b.p. 102—104°C/17.3 mbar (13 mm Hg); $n_D^{25}$ 1.4812.

In a run without a catalyst refluxing had to be continued for 2.25 hours to evolve the calculated amount ammonia. In both cases a thick precipitate was formed after the addition of the hexamethyldisilazane which disappeared as the silylation proceeded.

B. The above experiment was repeated whereby 117 mg (0.5 mmole) of sodium saccharinate 2 aq. were used instead of saccharin. The calculated amount of ammonia was evolved after refluxing for 40 minutes. Fractional distillation yielded 18.85 g (97.2%) of trimethylsilyl benzoate, b.p. 56—57°C/0.67 mbar (0.5 mm Hg); $n_D^{25}$ 1.4843.

Example 30

Preparation of trimethylsilyl trichloroacetate

A solution of 12.3 g (75 mmoles) of trichloroacetic acid in 30 ml of 1,2 - dichloroethane was added dropwise within 10 minutes at room temperature to a mixture of 34 mg (0.18 mmole) of saccharin and 31.3 ml (150 mmoles) of hexamethyldisilazane, while a stream of dry nitrogen was passed over the mixture. Thereby a precipitate was formed. Then the mixture was boiled under reflux for 1.5 hours, whereby the two-layer system present at the outset faded into a homogeneous solution and the calculated amount of ammonia was collected. Then dichloroethane was removed by distillation under normal pressure, whereby a small amount of a solid was separated in the condenser. Vacuum distillation of the residue yielded 14.94 g (84.9%) of trimethylsilyl trichloroacetate; b.p. 63—64°C/14.6 mbar (11 mm Hg); $n_D^{25}$ 1.4360.

Example 31

Preparation of trimethylsilyl 2-trimethylsilyloxybenzoate

To a mixture of 13.80 g (0.10 mole) of salicylic acid and 50 mg (0.10 mmole) of di - 4 - nitrophenyl N(4 - toluenesulphonyl)phosphoramidate, which mixture was heated to 130°C, 41.7 g (0.20 mole) of hexamethyldisilazane were added dropwise in 5 minutes. The ammonia evolved was led into water by means of a dry stream of nitrogen, which was passed over the reaction mixture. It was established by titration with 1N sulphuric acid that the calculated amount of ammonia had been evolved within 45 minutes. Fractionation under reduced pressure yielded 27.86 g (98.8%) of trimethylsilyl 2 - trimethylsilyloxybenzoate; b.p. 104—105°C/2 mbar (1.5 mm Hg); $n_D^{25}$ 1.4746.

Example 32

Preparation of ethyl trimethylsilyl malonate

To a solution of 22.9 g (0.173 mole) of ethyl hydrogen malonate and 30 mg (0.17 mmole) of saccharin in 25 ml of dichloromethane, which solution was boiling under reflux while a stream of dry nitrogen was passed over, 20.8 ml (0.10 mmole) of hexamethyldisilazane were added dropwise quickly. Thereby, a two-layer system was obtained which faded into a homogeneous solution as the reaction proceeded. By titration of the ammonia evolved it was established that the evolution of ammonia was completed after 1.5 hours. Boiling was continued for another 0.5 hour and then the mixture was fractionated under reduced pressure, yielding 34.1 g (96.6%) of ethyl trimethylsilyl malonate; b.p. 49.0—50.5°C/0.53 mbar (0.4 mm Hg); $n_D^{26}$ 1.4135.

Example 33

Preparation of trimethylsilyl 5-mercapto-1H-tetrazolyl-1-acetate

To 0.30 g (1.86 mmoles) of 5 - mercapto - 1H - tetrazolyl - 1 - acetic acid in 25 ml of 1,2 - dichloroethane, 6.0 mg (0.012 mmole) of tetraphenylimidodiphosphate were added and then while boiling under reflux, 0.5 ml (2.4 mmoles) of hexamethyldisilazane. After boiling under reflux for 1 hour, 1 equivalent of ammonia had been evolved. The mixture was then evaporated to dryness under reduced pressure and the residue was dried, yielding 0.45 g (105%) of trimethylsilyl 5 - mercapto - 1H - tetrazolyl - 1 - acetate; m.p. 130—133°C.

$^1H$ NMR ($CDCl_3$): 0.30 (s, 9H), 4.99 (s, 2H); 14.28 (s, 1H).

Example 34

Preparation of N,N'-bis(trimethylsilyl)urea

Hexamethyldisilazane (10 ml; 48 mmoles) was added to 2.4 g (40 mmoles) of urea and 73 mg (0.4 mmole) of saccharin in 15 ml of refluxing ethyl acetate. The evolution of ammonia started immediately and was completed after refluxing for 20 minutes, as was established by titration with 1N HCl. The volatile material was evaporated under vacuum and the residue was vacuum dried. There were obtained 8.06 g of N,N' - bis(trimethylsilyl)urea, m.p. 219—222°C (99%).

Without the addition of saccharin as a catalyst the evolution of ammonia is slow and the reaction has to be carried on for at least 24 hours to go to completion.

Example 35

Preparation of N-trimethylsilyl-trichloroacetamide

A mixture of 16.24 g (0.10 mole) of trichloroacetamide, 15 mg (0.08 mmole) of saccharin, 25 ml of toluene and 15 ml (0.07 mole) of hexamethyldisilazane was placed in a preheated oil bath (120°C) and refluxed for 30 minutes, after which evolution of ammonia was no longer detectable. The volatile materials were evaporated under vacuum and the residue was vacuum dried at 50°C. The crude N - trimethylsilyl - trichloroacetamide had m.p. 75—85°C and dissolved clearly in petroleum ether. Yield: 22.58 g (96.3%).

Example 36

Preparation of N-trimethylsilylbenzamide

To 15 ml of toluene were added saccharin (40 mg; 0.22 mmole) and benzamide (5.0 g; 41.3 mmoles) and the mixture obtained was heated to reflux. Hexamethyldisilazane (6.4 ml; 31 mmoles) was added and the ammonia liberated was led into water by means of a stream nitrogen led over the reaction mixture. Titration with 1N $H_2SO_4$ revealed that the calculated amount of ammonia was evolved in 15 minutes. Solvent and excess of hexamethyldisilazane were evaporated in vacuo, giving a residue of 8.04 g (101%) of N - trimethylsilylbenzamide, m.p. 111—114.5°C.

The experiment was repeated without the addition of saccharin and in that case it was found that 82% of the theoretical amount of ammonia was evolved in 15 minutes and 88% of it in 50 minutes.

Example 37

Preparation of N-trimethylsilyl-4-nitrobenzamide

A. 4-Nitrobenzamide (5.0 g; 30.1 mmoles) was treated with hexamethyldisilazane (4.7 ml of 93% purity; 21 mmoles) in 20 ml of refluxing butyl acetate in the presence of saccharin (50 mg; 0.27 mmole) by the method described in Example 18. The theoretical amount of ammonia was evolved during 15 minutes. Evaporation of the volatile materials in vacuo gave a pale brown residue of N - trimethylsilyl - 4 - nitrobenzamide (7.2 g; 100%), m.p. 130.5—134.5°C.

B. Hexamethyldisilazane (4.5 ml; 22 mmoles) was added to a refluxing mixture of 5.0 g (30.1 mmoles) of 4 - nitrobenzamide, 100 mg (0.5 mmole) of 1,8 - naphthalimide and 20 ml of butyl acetate. The calculated amount of ammonia was evolved after refluxing for 35 minutes.

The same experiment without catalyst was carried out and in that case only 16% of the theoretical amount of ammonia was evolved after refluxing for 15 minutes; after refluxing for 1 hour 83% of that amount was evolved.

Example 38

Preparation of N-trimethylsilyl-alpha,alpha-dimethylpropionamide

To 5.0 g (49.5 mmoles) of alpha,alpha-dimethylpropionamide and 10 mg (0.05 mmole) of saccharin in 15 ml of refluxing toluene were added during 15 minutes 7.7 ml (37 mmoles) of hexamethyldisilazane. After refluxing for 45 minutes the evolution of ammonia had stopped completely. Evaporation and drying in vacuo yielded 8.04 g (98%) of N - trimethylsilyl - alpha,alpha - dimethylpropionamide, m.p. 101—105.5°C. According to NMR analysis the purity of the product was 92%.

Example 39

Preparation of N-trimethylsilylacetamide

A mixture of 5.90 g (0.1 mole) of acetamide and 55 mg (0.3 mmole) of saccharin was heated to 130°C and 15.6 ml (0.075 mole) of hexamethyldisilazane were added during 3 minutes. By the method described in Example 1A it was found that the calculated amount of ammonia was evolved in 35 minutes after starting the addition of the hexamethyldisilazane. Refluxing was continued for 10 minutes, the excess of hexamethyldisilazane was evaporated under reduced pressure and the solid residue was dried under vacuum. There were obtained 12.80 g (97.7%) of N - trimethylsilylacetamide of more than 95% purity according to NMR analysis.

Example 40

Preparation of N-trimethylsilylurethane

Hexamethyldisilazane (15.6 ml; 0.075 mole) was added during 2 minutes to a refluxing mixture consisting of 8.9 g (0.1 mole) of urethane, 183 mg (1 mmole) of saccharin and 10 ml of toluene. The theoretical amount of ammonia was evolved in 30 minutes. Refluxing was continued for 15 minutes, the solvent and the excess of hexamethyldisilazane were removed under diminished pressure and the residue was vacuum distilled. There were obtained 15.6 g (96.9%) of N - trimethylsilylurethane, b.p. 73°C/16 mbar (12 mm Hg); $n_D^{25}$ 1.4268.

Example 41

Preparation of N,N'-bis(trimethylsilyl)-malonamide

A. 7.5 ml (36 mmoles) of hexamethyldisilazane were added to a refluxing mixture consisting of 3.06 g

(0.03 mole) of malonamide, 18.3 mg (0.1 mmole) of saccharin, 50 ml of ethyl acetate and 5 ml of pyridine. By titrating with 1N sulphuric acid of the ammonia evolved it was found that 30 mmoles of it were expelled after refluxing for one hour. Volatile material was vacuum evaporated and the residue, which crystallised upon standing, was dried in vacuo. There were obtained 7.32 g (99%) of N,N' - bis(trimethylsilyl)-malonamide, m.p. 72—76°C. NMR spectrum ($CDCl_3$): delta 0.24 (s, 18 H); 3.20 (s, 2 H); 6.5 (broad, 2H).

B. A similar run, using 30 ml of butyl acetate as the solvent, required refluxing for 15 minutes to evolve the calculated amount of ammonia. The melting point of the residue was 71—80°C. Yield 6.72 g (91%).

Example 42

Preparation of N-trimethylsilylcaprolactam

A mixture of 22.6 g (0.2 mole) of caprolactam, 0.73 g (4 mmoles) of saccharin and 40 ml (0.19 mole) of hexamethyldisilazane was refluxed for 3.5 hours, after which time ammonia evolution could no longer be detected. The dark brown reaction mixture was vacuum distilled to yield 21.64 g (58.5%) of N - trimethylsilylcaprolactam of b.p. 103—106°C/16 mbar (12 mm Hg).

Example 43

Preparation of trimethylsilylsaccharin

To a mixture of 1.83 g (10 mmoles) of saccharin, 10 mg (0.02 mmole) of di - 4 - nitrophenyl N - (4 - toluenesulphonyl)phosphoramidate and 20 ml of acetonitrile, which mixture was boiling under reflux while a stream of dry nitrogen was passed over, 2 ml (9.6 mmoles) of hexamethyldisilazane were added. The ammonia evolved was led into water by means of the nitrogen stream and titrated with 1N sulphuric acid. The calculated amount of ammonia had been evolved within 0.5 hour. The solvent and other volatile materials were removed by evaporation at a rotating film evaporator and then the residue was dried under vacuum, yielding 2.50 g (98%) of trimethylsilylsaccharin, m.p. 90—92°C.

$^1H$ NMR ($CCl_4$): 0.53 and 0.57 (two singlets, together 9H); 7.66—8.13 (m, 4H).

$^{13}C$ NMR ($CDCl_3$, 20 MHz, internal standard TMS): −1.2; −0.4; 1.8; 120.5; 121.4; 123.6; 124.8; 133.3; 133.8; 134.8.

Example 44

Preparation of N-trimethylsilylbenzenesulfonamide

Hexamethyldisilazane (15.6 ml; 75 mmoles) was added to a refluxing suspension of 15.72 g (0.1 mole) of benzenesulfonamide and 18 mg (0.1 mmole) of saccharin in 45 ml of ethyl acetate. A stream of nitrogen was led over the reaction mixture and passed through water in order to determine the amount of ammonia evolved. By titrating with 1N $H_2SO_4$ it was found that the calculated amount of ammonia was set free in 25 minutes. The residue of N - trimethylsilylbenzenesulfonamide obtained after evaporating the volatile materials in vacuo had m.p. 62—63°C.

Example 45

Preparation of N-trimethylsilylmethanesulfonamide

Hexamethyldisilazane (5.1 ml; 24.5 mmoles) was added to a refluxing mixture consisting of 3.0 g (31.6 mmoles) of methanesulfonamide, 20 mg (0.11 mmole) of saccharin and 15 ml of toluene. A stream of nitrogen was passed over the reaction mixture to expel the ammonia evolved, which was absorbed in water and titrated with 1N $H_2SO_4$. It was found that after refluxing for 20 minutes the calculated amount of ammonia was evolved. The solvent and the excess of hexamethyldisilazane were evaporated in vacuo. The solid residue was vacuum dried. The yield of N - trimethylsilylmethanesulfonamide was 5.22 g (99.5%), m.p. 69—74.5°C.

The experiment was repeated without the addition of saccharin. In that case the calculated amount of ammonia was evolved in 35 minutes. Workup as described above yielded 5.25 g (100%) of N - trimethylsilylmethanesulfonamide, m.p. 68—72.5°C.

Example 46

Preparation of N-(trimethylsilyl)thioacetamide

Hexamethyldisilazane (17.2 ml; 82 mmoles) was added during 10 minutes to a refluxing mixture consisting of 11.3 g (0.15 mole) of thioacetamide, 0.14 g (0.75 mmole) of saccharin and 50 ml of toluene. The ammonia produced in the reaction was passed by means of a stream of dry nitrogen into water and titrated with 1N HCl. It was found that after refluxing for 1.5 hours after the addition of the hexamethyldisilazane the calculated amount of ammonia was evolved. Toluene and the excess of hexamethyldisilazane were distilled off at normal pressure and the residue was vacuum distilled to yield 13.12 g (59.2%) of N - (trimethylsilyl)thioacetamide, b.p. 97—99°C/0.93 mbar (0.7 mm Hg).

Example 47

Preparation of N-trimethylsilyl diphenylphosphoramidate

Hexamethyldisilazane (3.2 ml; 15.4 mmoles) was added to 5.0 g (20.1 mmoles) of diphenylphosphoramidate and 36 mg (0.20 mmole) of saccharin in 35 ml of refluxing toluene. A stream of

nitrogen was led over the reaction mixture and the ammonia evolved was absorbed in water and titrated with 1N $H_2SO_4$. The calculated quantity of ammonia was liberated in 15 minutes. Refluxing was continued for 10 minutes and the solvent evaporated in vacuo. The N - trimethylsilyl diphenylphosphoramidate obtained (6.69 g) had m.p. 83—86.5°C.

The experiment was repeated without the addition of saccharin. After refluxing for 15 minutes 26% of the calculated quantity of ammonia was liberated and 69% after refluxing for 1 hour.

Example 48

Preparation of N,O,O-tris(trimethylsilyl)-DL-serine

To a suspension of 10.50 g (0.1 mole) of DL-serine in 30 ml of toluene were added 91 mg (0.5 mmole) of saccharin. The mixture was heated to reflux and 52.2 ml (0.25 mole) of hexamethyldisilazane were added. A stream of dry nitrogen was led over the reaction mixture and passed through water in order to determine the amount of ammonia evolved. By titrating with 1N $H_2SO_4$ it was found that three equivalents of ammonia were evolved in three hours (two equivalents were evolved after one hour). The toluene and the excess of hexamethyldisilazane were evaporated in vacuo and the residue was vacuum distilled to give 22.92 g (76.2%) of N,O,O - tris(trimethylsilyl) - DL - serine; b.p. 87—89°C/0.67—0.8 mbar (0.5—0.6 mm Hg); $n_D^{25}$ 1.4213.

Example 49

Preparation of trimethylsilyl d,l-alpha-trimethylsilylamino-propionate

A mixture of 8.90 g (0.1 mole) of d,l - alanine and 50 mg (0.1 mmole) of di - 4 - nitrophenyl N - 4 - toluenesulphonylphosphoramidate was placed in a preheated (140°C) oil bath and 41.6 ml (0.2 mole) of hexamethyldisilazane were added. After refluxing for 2 hours the calculated amount (0.1 mole) of ammonia was evolved as was established by leading it into water and titrating with 1N $H_2SO_4$.

The colourless solution was vacuum distilled to yield 20.72 g (88.9%) of trimethylsilyl dl - alpha - trimethylsilylaminopropionate, b.p. 78—81°C/24 mbar (18 mm Hg); $n_D^{25}$ 1.4145.

Example 50

Preparation of N-trimethylsilylsuccinimide

A. To a refluxing suspension consisting of a mixture of 50 ml of toluene and 19.80 g (0.20 mole) of succinimide, saccharin (458 mg; 2.5 mmoles) and hexamethyldisilazane (31.5 ml; 0.15 mole) were added and refluxing was continued for 2 hours. Fifteen minutes after the addition of the silylating agent a clear, light yellow solution was obtained, which turned brown when refluxing was continued. The reaction mixture was cooled, filtered and after evaporation of the solvent the residue was vacuum distilled. There were obtained 31.04 g (90.8%) of N - trimethylsilylsuccinimide with b.p. 86—88°C/1.7 mbar (1.3 mm Hg).

B. To a refluxing suspension of 9.90 g (0.1 mole) of succinimide and 0.24 g (0.5 mmole) of di - 4 - nitrophenyl N - 4 - toluenesulphonyl phosphoramidate in 50 ml of dichloromethane, were added dropwise in a few minutes 15.6 ml (0.075 mole) of hexamethyldisilazane. By means of a stream of nitrogen that was led over the reaction mixture the ammonia evolved was passed into water. The progress of the reaction was established by titration of the ammonia evolved. It was found that after boiling under reflux for 1.5 hours the production of ammonia was completed. Then 90% of the theoretical amount of ammonia had been evolved. Boiling was continued for another 2 hours and then the solvent was removed by distillation at normal pressure. Fractionation under reduced pressure yielded 15.36 g (89.8%) of N - trimethylsilylsuccinimide; b.p. 118—119°C/24 mbar (18 mm Hg), $n_D^{25}$ 1.4745.

Example 51

Preparation of N-trimethylsilylphthalimide

A mixture consisting of 36.8 g (0.25 mole) of phthalimide, 0.92 g (5 mmoles) of saccharin and 75 ml (0.36 mole) of hexamethyldisilazane was placed in an oil bath that was preheated to 120°C. The evolution of ammonia started immediately and a clear solution was obtained after 30 minutes. After that time the mixture was refluxed for 60 minutes. Volatile materials were evaporated under vacuum, 50 ml of petroleum ether (b.p. 80—110°C) were added and the mixture was evaporated again to dryness.

The nearly colourless residue had m.p. 66—68°C, which is in good agreement with literature data of N - trimethylsilylphthalimide. The alleged structure of the compound was also confirmed by its NMR spectrum. Yield: 54.89 g (100%).

Example 52

Preparation of N-trimethylsilylimidazole

To a mixture of 13.62 g (0.2 mole) of imidazole and 28 mg (0.15 mmoles) of saccharin, which was heated to 100°C 31.5 ml (0.15 mole) of hexamethyldisilazane were added dropwise in the course of 45 minutes. During this addition the bath temperature was raised from 100 to 140°C. After addition of the hexamethyldisilazane the mixture was stirred for 30 minutes at a bath temperature of 140°C. The excess of hexamethyldisilazane was evaporated under reduced pressure and the residue was vacuum distilled. There were obtained 22.25 g (79.5%) of N - trimethylsilylimidazole, b.p. 103—105°C/29.3 mbar (22 mm Hg), $n_D^{23.5}$ 1.4740.

Example 53

Preparation of 1,3-bis(trimethylsilyl)-5,5-dimethylhydantoin

Hexamethyldisilazane (80 ml; 0.38 mole) was added during 30 minutes to a refluxing suspension consisting of 50 ml of toluene, 40 mg (0.22 mmole) of saccharin and 38.45 g (0.30 mole) of 5,5 - dimethylhydantoin. The evolution of ammonia started immediately. When half of the hexamethyldisilazane had been added, all solids had gone into solution. After the addition of the hexamethyldisilazane refluxing was continued for 1 hour, the toluene was distilled off and the residue was vacuum dried at 45°C. There were obtained 79.0 g (97%) of 1,3 - bis(trimethylsilyl) - 5,5 - dimethyl - hydantoin, m.p. 46—49°C. According to NMR analysis the purity of the compound was 91%.

Example 54

Preparation of N-trimethylsilyl-2-oxazolidone

To 25 ml of toluene were added saccharin (10 mg; 0.05 mmole) and 2 - oxazolidone of 94% purity (10.0 g; 108 mmoles) and the mixture was heated to reflux. Hexamethyldisilazane (14.3 ml; 69 mmoles) was added during 10 minutes and refluxing was continued for 1 hour. The evolution of ammonia had stopped completely after that period of time. The solvent was evaporated in vacuo and the residue was vacuum distilled to give 14.6 g (85%) of N - trimethylsilyl - 2 - oxazolidone, b.p. 62°C/0.27 mbar (0.2 mm Hg); $n_D^{23}$ 1.4529.

Example 55

Preparation of 1-trimethylsilyl-1,2,4-1H-triazole

To a mixture consisting of 10.35 g (0.15 mole) of 1,2,4 - 1H - triazole and 127 mg (0.75 mmole) of saccharin, which was heated to 126°C were added 23.4 ml (0.11 mole) of hexamethyldisilazane. The evolution of ammonia started immediately. After refluxing for 30 minutes the calculated amount of ammonia was evolved as determined by the method described in Example 1A. Vacuum distillation yielded 19.37 g (91.6%) of 1 - trimethylsilyl - 1,2,4 - 1H - triazole, b.p. 76.5—78.0°C/16 mbar (12 mm Hg); $n_D^{25}$ 1.4592.

Example 56

Preparation of trimethylsilyl 6-aminopenicillanate

A. To a refluxing suspension consisting of 1.08 g (5.0 mmoles) of 6 - aminopenicillanic acid and 20 mg (0.11 mmole) of saccharin in 20 ml of dichloromethane, hexamethyldisilazane was added (1.0 ml; 4.8 mmole). After refluxing for 0.5 hour a substantially clear solution was obtained, indicating that the dichloromethane-insoluble 6 - aminopenicillanic acid was converted into the soluble trimethylsilyl ester. Omission of the saccharin prolonged the reaction time to 4 hours.

B. To a suspension consisting of 1.08 g (5.0 mmoles) of 6 - aminopenicillanic acid and 30 mg (0.11 mmole) of dimethyl N - trichloroacetylphosphoramidate in 20 ml of refluxing dichloromethane was added hexamethyldisilazane (1.0 ml; 4.8 mmoles). A clear solution, indicating that the dichloromethane-insoluble 6 - aminopenicillanic acid was converted into the soluble trimethylsilyl ester, was obtained after refluxing for 40 minutes.

C. A suspension consisting of 1.08 g (5.0 mmoles) of 6 - aminopenicillanic acid, 20 mg (0.11 mmole) of saccharin and 15 ml of chloroform (alcohol-free) was heated to reflux and hexamethyldisilazane (0.75 ml; 3.6 mmole) was added to it. After refluxing for 20 minutes a substantially clear solution was obtained, indicating that the dichloromethane-insoluble 6 - aminopenicillanic acid was converted into the soluble trimethylsilyl ester. Omission of the saccharin prolonged the reaction time to 1.5 hours.

D. To a suspension consisting of 1.08 g (5.0 mmoles) of 6 - aminopenicillanic acid and 53 mg (0.11 mmole) of di - 4 - nitrophenyl N - trichloroacetylphosphoramidate in 20 ml of refluxing dichloromethane was added 1.0 ml (4.8 mmoles) of hexamethyldisilazane. A clear solution, indicating that the dichloromethane-insoluble 6 - aminopenicillanic acid was converted into the soluble trimethylsilyl ester, was obtained after refluxing for 25 minutes.

Example 57

Preparation of trimethylsilyl 6-trimethylsilylaminopenicillanate

To a refluxing suspension consisting of 0.86 g (4 mmoles) of 6 - aminopenicillanic acid, 12 mg (0.07 mmole) of saccharin and 25 ml of chloroform was added hexamethyldisilazane (2.5 ml; 12 mmoles). After refluxing for 20 minutes a clear solution was obtained. After refluxing for 2 hours volatile material was evaporated under vacuum at a bath temperature of 40°C. The clear, colourless oil that remained was dissolved in 4 ml of dry carbon tetrachloride. From the NMR spectrum of this solution it could be concluded that trimethylsilyl 6 - trimethylsilylaminopenicillanate was present for 90%.

Example 58

Preparation of trimethylsilyl 6-D-(−)-alpha-aminophenylacetamido)penicillanate

6-(D-(−)-alpha-aminophenylacetamido)penicillanic acid (3.49 g; 10 mmoles) was suspended in 35 ml of dichloromethane and saccharin (92 mg; 0.5 mmole) was added. The mixture was heated to reflux and 1.55 ml (7.4 mmoles) of hexamethyldisilazane were added. A clear, colourless solution was obtained after

refluxing for 25 minutes, indicating that the dichloromethane-insoluble penicillanic acid derivative was converted into the soluble trimethylsilyl ester. A parallel run in which no saccharin was added gave a clear solution after refluxing for 50 minutes.

Example 59

Preparation of trimethylsilyl 6-(D-(−)-alpha-amino-p-trimethylsilyloxyphenylacetamido) penicillanate

To a refluxing suspension of 0.73 g (2 mmoles) of 6 - (D - (−) - alpha - amino - p - hydroxyphenylacetamido)penicillanic acid and 5 mg (0.027 mmole) of saccharin in 15 ml of dichloromethane was added 0.7 ml (3.4 mmoles) of hexamethyldisilazane. The clear solution that was obtained after refluxing for 20 minutes was refluxed for one hour more. The solvent was evaporated in vacuo and the residue was vacuum dried. The solid residue (1.15 g) was the O,O - bis(trimethylsilyl) derivative of the starting material according to the NMR spectrum.

In a parallel run without the addition of saccharin it took two and a half hours of refluxing to obtain a clear solution.

Example 60

Preparation of trimethylsilyl 6-trimethylsilylaminopenicillanate 1-oxide

In the same manner as described in Example 72 465 mg (2.0 mmoles) of 6 - aminopenicillanic acid 1 - oxide were silylated in 20 ml of chloroform with 1.6 ml (7.7 mmoles) of hexamethyldisilazane using 1.0 mg (0.002 mmole) of tetraphenyl imidodiphosphate as the catalyst. The calculated amount of ammonia was evolved in 1.5 hours. After boiling for 1.75 hours the mixture was evaporated to dryness at a rotating film evaporator and after addition of 10 ml of dry carbon tetrachloride evaporation to dryness was repeated. The residue (0.81 g) was dissolved in carbon tetrachloride.

$^1$H NMR: 0.11 (s, 9H); 0.33 (s, 9H); 1.17 (s, 3H); 1.65 (s, 3H); 2.36 (d, 1H, J 12.8 Hz); 4.45 (s, 1H); 4.45, 4.76 and 4.72 (dd and s, 2H, J 4.5 Hz).

Example 61

Preparation of trimethylsilyl 6-trimethylsilylaminopenicillanate 1,1-dioxide

In the same manner 506 mg (2.0 mmoles) of 6 - amino - penicillanic acid 1,1 - dioxide (containing 6% by weight of water) were silylated with 1.9 ml (9.1 mmoles) of hexamethyldisilazane in 20 ml of chloroform, using 1.0 mg (0.002 mmole) of di - 4 - nitrophenyl N - trichloroacetylphosphoramidate as the catalyst. The calculated amount of ammonia (3.6 mmoles) was evolved in 3.25 hours. The solvent was then removed under reduced pressure. By means of the $^1$H NMR spectrum of the residue (0.76 g) it was established that a complete disilylation had been effected.

$^1$H NMR ($CCl_4$): 0.14 (s, 9H); 0.34 (s, 9H); 1.33 (s, 3H); 1.53 (s, 3H); 2.27 (d, 1H, J 14 Hz); 4.28 (s, 1H); 4.47 (d, 1H, J 4.5 Hz); 4.60 and 4.85 (dd, 1H, J 4.5 and 14 Hz).

Example 62

Preparation of trimethylsilyl 7-amino-3-methyl-3-cephem-4-carboxylate

A. To a refluxing suspension of 1.07 g (5.0 mmoles) of 7 - amino - 3 - methyl - 3 - cephem - 4 - carboxylic acid in 20 ml of chloroform (p.a. quality stabilized with ethanol) were added hexamethyldisilazane (1.25 ml; 6 mmoles) and saccharin (20 mg; 0.11 mmole). A gentle stream of dry nitrogen led over the reaction mixture helped to expel the ammonia produced and maintained the system under anhydrous conditions. After refluxing for 25 minutes a clear, yellowish solution was obtained, indicating that the chloroform-insoluble carboxylic acid was converted into the soluble trimethylsilyl ester.

Omission of the saccharin in the reaction mixture prolonged the reaction time to 3.5 hours.

B. By the method described in Example 62A, a clear solution was obtained after refluxing a mixture consisting of 20 ml of dichloromethane (alcohol-free), 1.07 g (5.0 mmoles) of 7 - amino - 3 - methyl - 3 - cephem - 4 - carboxylic acid, 1.05 ml (5 mmoles) of hexamethyldisilazane and 20 mg (0.11 mmole) of saccharin for 10 minutes.

The same experiment with only 6.1 mg (0.03 mmole) of saccharin needed a 20 minutes' reflux to give a clear solution; without saccharin the reaction time was 2.5 hours.

C. To a refluxing suspension of 1.07 g (5.0 mmoles) of 7 - amino - 3 - methyl - 3 - cephem - 4 - carboxylic acid in 20 ml of dichloromethane, 20 mg (0.11 mmole) of saccharin and 0.75 ml (3.6 mmoles) of hexamethyldisilazane were added. After refluxing for 30 minutes a clear solution was obtained. A parallel run without saccharin took 3.5 hours to obtain a clear solution.

D. To a refluxing suspension of 1.50 g (7.0 mmoles) of 7 - amino - 3 - methyl - 3 - cephem - 4 - carboxylic acid in 20 ml of dichloromethane were added trichloroacetamide (82 mg; 0.5 mmole) and hexamethyldisilazane (1.05 ml; 5.0 mmoles). A clear solution was obtained after refluxing for 50 minutes.

Without trichloroacetamide added the time requiring to obtain a clear solution was 210 minutes.

E. To a refluxing suspension of 1.07 g (5 mmoles) of 7 - amino - 3 - methyl - 3 - cephem - 4 - carboxylic acid in 20 ml of dichloromethane were added sulfamide (10 mg; 0.1 mmole) and hexamethyldisilazane (0.75 ml; 3.6 mmoles). After refluxing for 2 hours a clear solution was obtained.

With omission of the sulfamide it took 210 minutes to obtain a clear solution.

F. To a refluxing mixture consisting of 25 ml of dichloromethane, 1.07 g (5 mmoles) of 7 - amino - 3 -

methyl - 3 - cephem - 4 - carboxylic acid and 26 mg (0.11 mmole) of N,N' - bis(trimethylsilyl)sulfamide was added hexamethyldisilazane (0.75 ml; 3.6 mmoles). After refluxing for 2 hours a clear solution was obtained.

The same experiment without the addition of N,N' - bis(trimethylsilyl)sulfamide needed a 210 minutes' reflux to get a clear solution.

G. By the method described in Example 62F the reaction took 35 minutes when N-benzoylbenzene-sulfonamide (41.4 mg; 0.15 mmole) was used as a catalyst.

H. By the method described in Example 62F the reaction took 135 minutes when N - (2 - methoxy - 1 - naphthoyl) - 4 - toluenesulfonamide (54.8 mg; 0.15 mmole) was used as a catalyst.

I. By the method described in Example 62F the reaction took 150 minutes when N - (2 - methoxy - 1 - naphthoyl)methanesulfonamide (42.8 mg; 0.16 mmole) was used as a catalyst.

J. Hexamethyldisilazane (528 mg; 3.28 mmoles) was added to a refluxing suspension of 1.08 g (5.0 mmoles) of 7 - amino - 3 - methyl - 3 - cephem - 4 - carboxylic acid, 10 mg (0.05 mmole) of saccharin and 20 ml of dichloromethane. The clear solution obtained after refluxing for one hour was evaporated to dryness and the residue was dried at room temperature under vacuum. The residue (1.48 g) was analyzed by means of quantitative NMR analysis using an internal standard technique. Thus it was found that the yield of trimethylsilyl 7 - amino - 3 - methyl - 3 - cephem - 4 - carboxylate, calculated on the trimethylsilyl ester peak, was 93%. Only a trace amount of trimethylsilyl 3 - methyl - 7 - trimethylsilylamino - 3 - cephem - 4 - carboxylate could be detected in the NMR spectrum.

Example 63

Preparation of trimethylsilyl 7-trimethylsilylamino-3-methyl-3-cephem-4-carboxylate

A. To 40 ml of chloroform (pro analyse quality containing ethanol) was added di - 4 - nitrophenyl N - 4 - toluenesulphonylphosphoramidate (12 mg; 0.024 mmole) and the mixture was refluxed for half an hour to destroy any active hydrogen atoms-containing impurities present. The ammonia evolved during this period was driven off by leading a gentle stream of dry nitrogen over the mixture. Then 1.07 g (5.0 mmoles) of 7 - amino - 3 - methyl - 3 - cephem - 4 - carboxylic acid was added and refluxing was continued under the same conditions, but the stream of nitrogen now was led into water to absorb the ammonia evolved, which was titrated with 0.1N sulphuric acid. It was found that a clear solution was obtained after refluxing for 10 minutes (25 ml of the sulphuric acid solution was used then) and that 50 ml of the sulphuric acid solution, indicative of complete disilylation, was used after refluxing for 35 minutes.

B. A mixture consisting of 0.80 g (3.7 mmoles) of 7 - amino - 3 - methyl - 3 - cephem - 4 - carboxylic acid, 1.87 ml (11.6 mmoles) of hexamethyldisilazane, 8.8 mg (0.048 mmole) of saccharin and 20 ml of chloroform was heated to reflux. A clear solution was obtained after 10 minutes. After refluxing for 2 hours volatile material was evaporated under vacuum (bath temperature 50°C). The solid residue was dissolved in 5 ml of carbon tetrachloride. From the NMR spectrum of this solution it could be concluded that the N,O - disilylated derivative was present for 80% (by comparing the ratio of the N - trimethylsilyl and O - trimethylsilyl signals).

Example 64

Preparation of trimethylsilyl 3-methyl-7-phenylacetamido-3-cephem-4-carboxylate 1-oxide

A. Hexamethyldisilazane (1.25 ml; 6.0 mmoles) was added to a refluxing suspension of 1.4 g (4 mmoles) of 3 - methyl - 7 - phenylacetamido - 3 - cephem - 4 - carboxylic acid 1 - oxide in 25 ml of dichloromethane. A clear solution, being indicative of complete silylation, was obtained after refluxing for 30 minutes.

B. The experiment was repeated with 13 mg (0.04 mmole) of ditosylamine added as a catalyst. A clear solution was obtained after refluxing for 8 minutes.

C. Using 74.8 mg (0.66 mmole) of 2,2,2 - trifluoroacetamide as a catalyst a clear solution was obtained after refluxing for 15 minutes.

D. A mixture consisting of 1.083 g (3.1 mmoles) of 3 - methyl - 7 - phenylacetamido - 3 - cephem - 4 - carboxylic acid 1 - oxide, 42 mg (0.15 mmoles) of N - benzoyl - 4 - toluenesulfonamide and 25 ml of dichloromethane was heated to reflux. Hexamethyldisilazane (0.57 ml; 2.73 mmoles) was added and refluxing was continued for 5 minutes, after which time a clear, slightly brown solution was obtained.

A parallel run in which the catalyst was omitted gave a clear solution after refluxing for 45 minutes.

E. Hexamethyldisilazane (0.5 ml; 2.4 mmoles) was added to a refluxing mixture of 1.04 g (3.0 mmoles) of 3 - methyl - 7 - phenylacetamido - 3 - cephem - 4 - carboxylic acid 1 - oxide, 14.5 mg (0.3 mmole) of formamide and 30 ml of dichloromethane. A clear solution was obtained after refluxing for 45 minutes.

In a parallel run in which the formamide was omitted, refluxing for 75 minutes was necessary to obtain a clear solution.

Example 65

Preparation of trimethylsilyl 7-phenylacetamido-3-methyl-3-cephem-4-carboxylate 1-alpha-oxide

The title compound was prepared by boiling under reflux during 30 minutes a mixture consisting of 0.17 g (0.5 mmole) of 7 - phenylacetamido - 3 - methyl - 3 - cephem - 4 - carboxylic acid 1 - alpha -

oxide, 0.88 mg (0.005 mmole) of saccharin, 0.065 ml (0.31 mmole) of hexamethyldisilazane and 5 ml of deuterochloroform. According to the $^{1}H$ NMR spectrum the product had been formed quantatively.

$^{1}H$ NMR ($CDCl_3$): 0.33 (s, 9H); 2.18 (s, 3H); 3.33 and 3.98 (ABq, 2H, J 16.5 Hz); 3.58 (s, 2H); 4.47 (d, 1H, J 4.5 Hz); 5.29 and 5.42 (dd, 1H, J 4.5 and 8 Hz); 7.13 (d, 1H, J 8 Hz); 7.29 (s, 5H).

Example 66

Preparation of trimethylsilyl 7-formamido-3-methyl-3-cephem-4-carboxylate 1-oxide

To a refluxing boiling suspension consisting of 1.34 g (5.0 mmoles) of 7 - formamido - 3 - methyl - 3 - cephem - 4 - carboxylic acid 1 - oxide (96%), 5 mg (0.01 mmole) of tetraphenyl imidodiphosphate and 50 ml of dichloromethane, 0.62 ml (3.0 mmoles) of hexamethyldisilazane was added. The calculated amount of ammonia was evolved in 80 minutes. After evaporation under reduced pressure of the volatile materials and drying under vacuum at room temperature, there was obtained 1.63 g (99%) of trimethylsilyl 7 - formamido - 3 - methyl - 3 - cephem - 4 - carboxylate 1 - oxide as a solid of light-yellow colour.

The characterization of the product was carried out as follows: a mixture consisting of 106 mg of 7 - formamido - 3 - methyl - 3 - cephem - 4 - carboxylic acid 1 - oxide, 0.5 mg of saccharin, 3 ml of deuterochloroform and 0.05 ml of hexamethyldisilazane was boiled under reflux during 1 hour. From the $^{1}H$ NMR spectrum of the solution thus prepared it was established from the integration ratio between the trimethyl signal and the 7-proton signal that the silylation had proceeded quantatively.

$^{1}H$ NMR ($CDCl_3$): 0.32 (s, 9H); 2.16 (s, 3H); 3.23 and 3.68 (ABq, 2H, J 18 Hz); 4.53 (d, 1H, J 4,5 Hz); 5.97 and 6.11 (dd, 1H, J 4.5 and 9.8 Hz); 7.20 (d, 1H, J 9.8 Hz); 8.31 (s, 1H).

Example 67

Preparation of trimethylsilyl 7 - phenylacetamido - 3 - (1 - methyl - 1H - tetrazolyl - 5 - thio)methyl - 3 - cephem - 4 - carboxylate 1 - oxide

In the same manner as described in Example 75 115 mg (0.25 mmole) of 7 - phenylacetamido - 3 - (1 - methyl - 1H - tetrazolyl - 5 - thio)methyl - 3 - cephem - 4 - carboxylic acid 1 - oxide in 2 ml of deuterochloroform were silylated in 1.5 hours, with 0.10 ml (0.48 mmole) of hexamethyldisilazane, using 0.048 mg (0.00026 mmole) of saccharin as the catalyst.

$^{1}H$ NMR ($CDCl_3$): 0.33 (s, 9H); 3.54 (s, 2H); 3.25, 3.58, 3.88, 4.21 (ABq, 2H, J 19 Hz); 3.83 (s, 3H); 4.06, 4.29, 4.43, 4.66 (ABq, 2H, J 13 Hz); 4.44 (d, 1H, J 4.5 Hz); 5.99 (dd, 1H, J 4.5 and 10 Hz); 6.82 (d, 1H, J 10 Hz); 7.27 (s, 5H).

Example 68

Bis(trimethylsilylation of 7 - phenylacetamido - 3 - (1H - 1,2,3 - triazolyl - 5 - thio)methyl - 3 - cephem - 4 - carboxylic acid 1 - oxide

In the same manner as described in Example 76 130 mg (0.29 mmole) of 7 - phenylacetamido - 3 - (1H - 1,2,3 - triazolyl - 5 - thio)methyl - 3 - cephem - 4 - carboxylic acid 1 - oxide were converted into its bis(trimethylsilyl)derivative by boiling under reflux in 2 ml of deuterochloroform during 1 hour with 0.10 ml (0.48 mmole) of hexamethyldisilazane in the presence of 1.0 mg (0.002 mmole) of di - 4 - nitrophenyl N - trichloroacetylphosphoramidate as the catalyst. From the NMR spectrum it could be concluded that the product consisted of a mixture of two isomers differing as to the position of the trimethylsilyl group in the triazole ring.

$^{1}H$ NMR ($CDCl_3$): 0.25 and 0.28 (2s, together 9H); 0.49 and 0.53 (2s, together 9 H); about 3.2—4.7 (m, 7H); 5.95 (dd, 1H, J 4.5 and 9 Hz); 6.82 and 6.98 (2d, together 1H, in both J 9 Hz); 7.28 (s, 5H); 7.65 and 7.85 (2s, together 1H).

Example 69

Preparation of trimethylsilyl 3 - acetoxymethyl - 7 - amino - 3 - cephem - 4 - carboxylate

To a refluxing suspension of 0.82 g (3.0 mmoles) of 3 - acetoxy - methyl - 7 - amino - 3 - cephem - 4 - carboxylic acid in 15 ml of dichloromethane, saccharin (11 mg; 0.06 mmole) and hexamethyldisilazane (0.63 ml; 3.0 mmoles), were added. A clear solution, indicating that the chloroform-insoluble carboxylic acid was converted into the soluble trimethylsilyl ester, was obtained after refluxing for 10 minutes.

The same experiment without the addition of saccharin was also carried out. In that case refluxing had to be continued for 50 minutes to obtain a clear solution.

Example 70

Preparation of trimethylsilyl 7 - trimethylsilylamino - 3 - acetoxy - methyl - 3 - cephem - 4 - carboxylate

To a refluxing suspension consisting of 1.08 g (4 mmoles) of 3 - acetoxymethyl - 7 - amino - 3 - cephem - 4 - carboxylic acid, 15 mg (0.08 mmole) of saccharin and 25 ml of chloroform was added hexamethyldisilazane (2.5 ml; 12 mmoles). The mixture, which became clear after 10 minutes, was refluxed for 2 hours.

After evaporation of the solvent and the excess of hexamethyldisilazane under vacuum at a bath temperature of 40°C, a brown, oily residue was obtained. This was dissolved in 4 ml of dry carbon tetrachloride, filtered and subjected to NMR analysis. The trimethylsilyl 7 - trimethylsilylamino - 3 - acetoxymethyl - 3 - cephem - 4 - carboxylate appeared to be present for at least 80%.

Example 71

Preparation of trimethylsilyl 7 - trimethylsilylamino - 3 - (5 - methyl - 1,3,4 - thiadiazolyl - 2 - thio)methyl - 3 - cephem - 4 - carboxylate

A mixture consisting of 0.85 g (2.5 mmoles) of 7 - amino - 3 - (5 - methyl - 1,3,4 - thiadiazolyl - 2 - thio)methyl - 3 - cephem - 4 - carboxylic acid, 12 mg (0.07 mmole) of saccharin and 25 ml of chloroform was heated to reflux and hexamethyldisilazane (1.6 ml; 7.7 mmoles) was added to it. A clear solution was obtained instantaneously. After refluxing for 2 hours with volatile material was evaporated under reduced pressure and the clear, viscous residue was dissolved in 4 ml of dry carbon tetrachloride. From the NMR spectrum of this solution it could be concluded that trimethylsilyl 7 - trimethylsilylamino - 3 - (5 - methyl - 1,3,4 - thiadiazolyl - 2 - thio)methyl - 3 - cephem - 4 - carboxylate was formed for at least 80%.

Example 72

Preparation of trimethylsilyl 7 - trimethylsilylamino - 3 - (1 - trimethylsilyl - 1H - 1,2,3 - triazolyl - 5 - thio)methyl - 3 - cephem - 4 - carboxylate

To 20 ml of chloroform were added 1.65 ml (80 mmoles) of hexamethyldisilazane and 1.0 mg (0.002 mmole) of di - 4 - nitrophenyl N - (4 - toluenesulphonyl)phosphoramidate. The mixture was then refluxed till no more ammonia was evolved which was established by leading the ammonia into water by means of a stream of dry nitrogen which was passed over the reaction mixture and titration with 1N sulphuric acid. In 1.5 hours 2.6 mmoles of ammonia were evolved. Then 0.632 g (2 mmoles) of 7 - amino - 3 - (1H - 1,2,3 - triazolyl - 5 - thio)methyl - 3 - cephem - 4 - carboxylic acid was added to the mixture and boiling was continued under the same conditions till no more ammonia came free. In 1.75 hours 3 mmoles of ammonia were collected.

After evaporating to dryness under reduced pressure 5 ml of carbon tetrachloride were added and then the evaporation to dryness was repeated. The residue (1.13 g) was dissolved in dry carbon tetrachloride.

$^1$H NMR: 0.11 (s, 9H); 0.28 (s, 9H); 0.52 (s, 9H); 1.41 (d, 1H, J 12 Hz); 3.40 and 3.80 (ABq, 2H, J 18 Hz); 3.75 and 4.29 (ABq, 2H, J 13.5 Hz); 4.49 and 4.75, 4.73 (dd and s, 2H, J 4.8 Hz); 7.57 (s, 1H).

IR ($CCl_4$): 3400, 1795, 1720, 1380, 1270, 865 $cm^{-1}$.

Example 73

Preparation of trimethylsilyl 7 - trimethylsilylamino - 3 - (1 - methyl - 1H - tetrazolyl - 5 - thio)methyl - 3 - cephem - 4 - carboxylate.

In the same manner as described in Example 72 1.64 g (4.6 mmoles) of 7 - amino - 3 - (1 - methyl - 1H - tetrazolyl - 5 - thio)methyl - 3 - cephem - 4 - carboxylic acid (with a content of 91%) in 30 ml of chloroform were silylated in 2 hours with 3.0 ml (14.4 mmoles) of hexamethyldisilazane, using 5.0 mg (0.01 mmole) of di - 4 - nitrophenyl N - trichloroacetylphosphoramidate as the catalyst. The mixture was concentrated by evaporation at a rotating film evaporator to a foam which was then dried under vacuum, yielding 2.21 g (103%) of the title compound.

$^1$H NMR ($CCl_4$): 0.11 (s, 9H); 0.34 (s, 9H); 1.43 (d, 1H, J 12 Hz); 3.66 (s, 2H); 3.90 (s, 3H); 4.14 and 4.51 (ABq, 2H, J 13.5 Hz); 4.58, 4.83 and 4.82 (dd and s, 2H, J 4.5 Hz).

Example 74

Preparation of trimethylsilyl 7 - D - (O - trimethylsilyl)mandelamido - 3 - (1 - methyl - 1H - tetrazolyl - 5 - thio)methyl - 3 - cephem - 4 - carboxylate

A suspension consisting of 3.23 g (6.3 mmoles) of 7 - D - (−) - mandelamido - 3 - (1 - methyl - 1H - tetrazolyl - 5 - thio)methyl - 3 - cephem - 4 - carboxylic acid (90%; cefamandole), 10.0 mg (0.02 mmole) of di - 4 - nitrophenyl N - (4 - toluenesulphonyl)phosphoramidate, 1.90 ml (9.1 mmoles) of hexamethyldisilazane and 30 ml of dichloromethane was boiled under reflux. The ammonia evolved was led into water by means of a dry stream of nitrogen which was passed over the reaction mixture. It was established by titration with 1N sulphuric acid that the evolution of ammonia was completed after boiling for 0.5 hour. Thereby 65 ml of 1N sulphuric acid had been used. The solid remaining after evaporation to dryness under reduced pressure was washed with a mixture of 20 ml of petroleum ether and 5 ml of ethyl acetate, yielding 3.1 g (81%) of the title compound; the $^1$H NMR spectrum was in conformity with that known from literature.

Example 75

Preparation of trimethylsilyl 7 - phenylacetamido - 3 - (1 - methyl - 1H - tetrazolyl - 5 - thio)methyl - 3 - cephem - 4 - carboxylate

A solution of saccharin in dichloromethane 1 ml, containing 0.048 mg (0.00026 mmole) of saccharin, was put into a 25 ml roundbottom flask and then the solvent was evaporated under reduced pressure. Subsequently, 113 mg (0.025 mmole) of 7 - phenylacetamido - 3 - (1 - methyl - 1H - tetrazolyl - 5 - thio)methyl - 3 - cephem - 4 - carboxylic acid were weighed out in the vessel and then 2 ml of deuterochloroform and 0.05 ml (0.25 mmole) of hexamethyldisilazane were added. While a gentle stream of dry nitrogen was passed through the mixture, it was stirred at room temperature. After 10 minutes an almost clear solution was obtained. After stirring for 1 hour, a NMR spectrum of the clear solution was recorded, from which it appeared that the silylation had proceeded quantatively.

$^1$H NMR ($CDCl_3$): 0.31 (s, 9H); 3.58 (s, 2H); 3.64 (s, 2H) 3.86 (s, 3H); 4.33 (s, 2H); 4.88 (d, 1H, J 4.5 Hz); 5.78 (dd, 1H, J 4.5 and 9 Hz); 6.22 (d, 1H, J 9 Hz); 7.27 (s, 5H).

Example 76

Preparation of trimethylsilyl 7 - phenylacetamido - 3 - (1 - trimethylsilyl - 1H - 1,2,3 - triazolyl - 5 - thio)methyl - 3 - cephem - 4 - carboxylate

In a 25 ml roundbottom flask 2 ml of a solution of di - 4 - nitrophenyl N - trichloroacetyl-phosporamidate, containing 0.25 mg (0.0005 mmole) of said substance, were evaporated to dryness under reduced pressure. Subsequently, 103 mg (0.25 mmole) of 7 - phenylacetamido - 3 - (1H - 1,2,3 - triazolyl - 5 - thio)methyl - 3 - cephem - 4 - carboxylic acid were weighed out in the vessel and 2 ml of deuterochloroform were added. Then, while boiling under reflux 0.10 ml (0.48 mmole) of hexamethyldisilazane was added. Boiling was continued for 45 minutes and then the mixture was evaporated to dryness under reduced pressure, yielding 144 mg of the title compound.

$^1$H NMR ($CDCl_3$): 0.28 (s, 9H); 0.53 (s, 9H); 3.23, 3.53, 3.64, 3.95 (ABq, 2H, J 18 Hz); 3.61 (s, 2H); 3.68, 3.90, 4.15, 4.39 (ABq, 2H, J 13 Hz); 4.88 (d, 1H, J 4.5 Hz); 5.78 (dd, 1H, J 4.5 and 9 Hz); 6.53 (d, 1H, J 9 Hz); 7.33 (s, 5H); 7.66 (s, 1H).

Example 77

Preparation of trimethylsilyl 7 - phenylacetamido - 3 - methyl - 3 - cephem - 4 - carboxylate 1 - oxide

142 mg (0.41 mmole) of 7 - phenylacetamido - 3 - methyl - 3 - cephem - 4 - carboxylic acid 1 - oxide and 0.5 mg (0.003 mmole) of saccharin were suspended in 3 ml of deuterochloroform. 9 - Methylanthracene (57 mg; 0.297 mmole) was added as an internal reference for quantitative NMR analysis. Hexamethyldisilazane (0.05 ml; 0.24 mmole) was added and the mixture was refluxed for 10 minutes. The clear, pale yellow solution obtained was cooled to room temperature.

From the NMR spectrum of this solution it was calculated, by comparing the integration ratios of the trimethylsilyl ester peak of the trimethylsilyl 7 - phenylacetamido - 3 - methyl - 3 - cephem - 4 - carboxylate 1 - oxide formed and the methyl peak of the 9 - methylanthracene added that the yield of trimethylsilyl 7 - phenylacetamido - 3 - methyl - 3 - cephem - 4 - carboxylate 1 - oxide was 97%.

Example 78

Preparation of trimethylsilyl 7 - trimethylsilylamino - 3 - methyl - 3 - cephem - 4 - carboxylate 1 - beta - oxide and 1 - alpha - oxide

A. To a suspension consisting of 122 mg (0.53 mmole) of 7 - amino - 3 - methyl - 3 - cephem - 4 - carboxylic acid 1 - beta - oxide, 0.5 mg (0.001 mmole) of di - 4 - nitrophenyl N - (4 - toluenesulphonyl)-phosphoramidate and 2 ml of dichloromethane, was added with stirring 0.2 ml (0.96 mmole) of hexamethyldisilazane. After boiling under reflux for 6 minutes while passing a dry stream of nitrogen over reaction mixture, a clear solution was obtained. After boiling 40 minutes the mixture was evaporated to dryness at a rotating film evaporator. The solid residue was dried under vacuum at room temperature. There were obtained 208 mg of trimethylsilyl 7 - trimethylsilylamino - 3 - methyl - 3 - cephem - 4 - carboxylate 1 - beta - oxide.

$^1$H NMR ($CDCl_3$): 0.14 (s, 9H); 0.36 (s, 9H); 1.99 (d, 1H, J 14 Hz); 2.16 (s, 3H); 3.19, 3.60 (Aq, 2H, J 18 Hz); 4.34 (d, 1H, J 4.5 Hz); 4.62, 4.86 (dd, 1H, J 4.5 and 14 Hz).

B. In the same manner trimethylsilyl 7 - trimethylsilylamino - 3 - methyl - 3 - cephem - 4 - carboxylate 1 - alpha - oxide was obtained.

$^1$H NMR ($CDCl_3$): 0.17 (s, 9H); 0.34 (s, 9H); 1.54 (d, 1H, J 13 Hz); 2.13 (s, 3H); 3.37 and 3.97 (ABq, 2H, J 17 Hz); 4.44 (d, 1H, H 4.5 Hz); 4.80 and 5.02 (dd, 1H, J 4.5 and 13 Hz).

Example 79

Preparation of 1-trimethylsilyl-2-phenylhydrazine

90 mg (0.5 mmole) of saccharin were added to 10.8 g (0.1 mole) of phenylhydrazine and the mixture obtained was heated to 130°C. Hexamethyldisilazane (15.6 ml; 0.075 mole) was added and refluxing was continued for 2.5 hours. By the method described in Example 1A it was established that after refluxing for two hours the calculated amount of ammonia was evolved. Vacuum distillation of the mixture yielded 16.15 g (89.7%) of 1 - trimethylsilyl - 2 - phenylhydrazine, b.p. 112—116°C/14.6 mbar (11 mm Hg); $n_D^{25}$ 1.5241.

Example 80

Preparation of N-trimethylsilyloxysuccinimide

A mixture of 5.60 g (47.2 mmoles) of N - hydroxysuccinimide (purity 97%) and 46 mg (0.25 mmole) of saccharin was heated to 130°C and 7.8 ml (37.5 mmoles) of hexamethyldisilazane were added. A fierce evolation of ammonia started immediately and was completed 15 minutes after the beginning of the addition of hexamethyldisilazane. The mixture was cooled, the excess of hexamethyldisilazane was distilled off at reduced pressure and the residue was fractionated to yield 7.70 g (87.2%) of N - trimethylsilyloxysuccinimide, b.p. 109—110°C/0.53 mbar (0.4 mm Hg), m.p. 55—57°C.

Example 81

Preparation of N-benzoyl-N,O-bis(trimethylsilyl)hydroxylamine

To a mixture of 1.21 g (8.8 mmoles) of benzohydroxamic acid, 5 mg (0.01 mmole) of di - 4 - nitrophenyl N - (4 - toluenesulphonyl)phosphoramidate and 10 ml of dichloromethane, which mixture

was boiling under reflux, 4.2 ml (20.2 mmoles) of hexamethyldisilazane were added dropwise quickly, whereby a precipitate was formed. After boiling under reflux for 15 minutes the evolution of ammonia had come to an end and a clear solution was obtained. The volatile materials were removed by evaporation under reduced pressure, yielding 2.35 g (95%) of N - benzoyl - N,O - bis(trimethylsilyl)hydroxylamine.

$^1H$ NMR ($CCl_4$): 0.26 (s, 9H); 0.30 (s, 9H); 7.2—7.4 (m, 3H); 7.6—7.8 (m, 2H).

Example 82

Preparation of N-trimethylsilyloxy-4-toluenesulfonamide

To a mixture consisting of 0.94 g (5.0 mmoles) of N - hydroxy - 4 - toluenesulfonamide, 5 mg (0.01 mmole) of tetraphenylimidodiphosphate and 20 ml of dichloromethane, which mixture was boiling under reflux, hexamethyldisilazane (1.0 ml; 4.8 mmoles) was added. By titration of the ammonia evolved with 0.1 N sulphuric acid it was established that the evolution of ammonia came to an end after boiling for 50 minutes. At that time 2.5 mmoles of ammonia had been collected. The reaction mixture was evaporated to dryness, yielding 1.29 g (100%) of N - trimethylsilyloxy - 4 - toluenesulfonamide, m.p. 87—90°C.

$^1H$ NMR ($CCl_4$): 0.17 (s, 9H); 2.43 (s, 3H); 6.90 (s, 1H); 7.20, 7.34, 7.69, 7.83 (q, 4H).

Example 83

Preparation of 1-trimethylsilyloxycyclohexen-3-one

8.4 g (72 mmoles) of cyclohexanedione-1,3 (purity 96%) and 70 mg (0.38 mmole) of saccharin were mixed with 60 ml (288 mmoles) of hexamethyldisilazane. The mixture was placed in a preheated oil bath and refluxed. By titrating the ammonia liberated during the reaction it was found that the calculated amount was expelled in 50 minutes. Refluxing was then continued for 10 minutes, the excess of hexamethyldisilazane was evaporated in vacuo and the residue was vacuum distilled. There were obtained 10.67 g (80.5%) of 1 - trimethylsilyloxycyclohexen - 3 - one, b.p. 119—121°C/2.7 mbar (2.0 mm Hg).

Example 84

Preparation of ethyl 3-trimethylsilyloxy-2-butenoate

A mixture consisting of 9.75 g (75 mmoles) of ethyl acetoacetate and 70 mg (0.38 mmole) of saccharin was heated to 130°C in an oil bath. Hexamethyldisilazane (60 ml; 288 mmoles) was added and the mixture was refluxed for 1.5 hours. The excess of hexamethyldisilazane was distilled off at reduced pressure and the residue was vacuum distilled to give 12.71 g (84%) of ethyl 3 - trimethylsilyloxy - 2 - butenoate; b.p. 102—104°C/21.3 mbar (16 mm Hg).

Example 85

Preparation of 1-trimethylsilyloxybutane

To a solution of one of the catalyst mentioned in the table below in 15 ml of dichloromethane, a solution of 1.48 g (20.0 mmoles) of 1-butanol in 10 ml of dichloromethane was added. This mixture was heated to reflux and 2.50 ml (12.0 mmoles) of hexamethyldisilazane were added. The time (t) in which half of the calculated amount of ammonia was evolved, was measured. Further details are to be found in the following table.

| Catalyst | Mol-% of catalyst | t (minutes) |
|---|---|---|
| none | | 42 |
| di-4-nitrophenyl N-(dimethylaminosulphonyl)phosphoramidate | 0.5 | 2 |
| diisopropyl N-(dichloroacetyl)phosphoramidate | 0.5 | 22 |
| di-o-chlorophenyl N-(4-chlorophenylsulphonyl)phosphoramidate | 1.0 | 17 |
| N,N-dimethylsulfamide | 5.0 | 15 |
| silylated saccharin | 0.1 | 4 |
| N-(1-naphthoyl)-4-toluenesulphonamide | 1.0 | 8 |
| N-(2-methoxybenzoyl)-4-toluenesulphonamide | 5.0 | 22 |

Example 86

Preparation of N,N-dimethyl-N'-trimethylsilylsulfamide

To a refluxing mixture of 2.48 g (20 mmoles) of N,N - dimethylsulfamide, 9 mg (0.05 mmole) of saccharin and 30 ml of toluene, hexamethyldisilazane (3 ml; 14.4 mmoles) was added. By the method described hereinbefore it was established that the calculated amount of ammonia had been evolved after

refluxing for one hour. Volatile materials were then evaporated under reduced pressure and the residue was dried at room temperature, yielding 3.96 g (101%) of N,N - dimethyl - N′ - trimethylsilylsulfamide, m.p. 83—86°C.

Example 87

Preparation of trimethylsilyl 2-(trimethylsilyl)thioacetate

To a refluxing solution of 1.20 g (13.0 mmoles) of mercaptoacetic acid and 18 mg (0.036 mmole) of di - 4 - nitrophenyl N - (4 - toluenesulphonyl)phosphoramidate in 10 ml of toluene, hexamethyldisilazane (4.0 ml; 19.2 mmoles) was added. By the method described hereinbefore it was established that the calculated amount of ammonia (13 mmoles) had been evolved after refluxing for 25 minutes. Volatile materials were then evaporated at the rotating film evaporator and the residue was kept under vacuum for 0.5 hour. There were obtained 2.87 g (93.4%) of trimethylsilyl 2 - (trimethylsilyl)thioacetate.

$^1H$ NMR ($CCl_4$): 0.28 (s, 9H); 0.32 (s, 9H); 3.01 (s, 2H).

**Claims**

1. Process for the trimethylsilylation of organic compounds carrying one or more active hydrogen atoms with hexamethyldisilazane, characterized in that the trimethylsilylation is effected in the presence of 0.001 to 10 mol % of a catalyst of the formula

X—NH—Y

wherein

a) X and Y are the same or different and each represents an electron-withdrawing group selected from

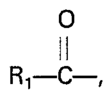, 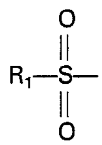 and 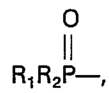,

in which $R_1$ and $R_2$ are the same or different, and each represents an alkyl group which may be substituted by one or more halogen atoms, an aryl group which may be substituted by one or more halogen atoms, alkyl, alkoxy or nitro groups, an alkoxy group, an aryloxy group which may be substituted by a halogen atom, an alkyl group or a nitro group, or a group $R_3R_4N$ in which $R_3$ and $R_4$ are the same or different and each represents a hydrogen atom, a trialkylsilyl group or an alkyl group,

b) X is an electron-withdrawing group as defined under a) and Y is a hydrogen atom or a trialkylsilyl group or,

c) X and Y together represent an electron-withdrawing group —A—Z—B— forming a cyclic system with the nitrogen atom, in which A represents a group

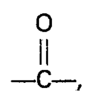,

B represents a group

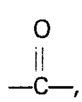, $—SO_2—$, —S—, 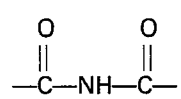

or a group

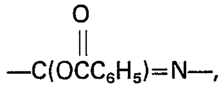,

and Z represents an alkylene, alkenylene or arylene group, which groups may be substituted by one or more halogen atoms or alkyl groups.

2. Process according to claim 1, wherein the electron-withdrawing groups are represented by the formulas:

[I] 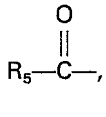,

wherein $R_5$ represents an alkyl group which may be substituted by one or more halogen atoms, or an aryl group, which may be substituted by one or more alkoxy or nitro groups,

[II] 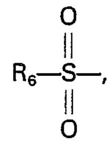

wherein $R_6$ represents a methyl group or an aryl group which may be substituted by one or more halogen atoms or methyl groups, or $R_6$ represents a group $R_7R_8N$— wherein $R_7$ and $R_8$ are the same or different and each represents a hydrogen atom, a trialkylsilyl group or an alkyl group,

[III] 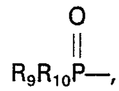

wherein $R_9$ and $R_{10}$ are the same or different and each represents an alkoxy group or an aryloxy group which may be substituted by a halogen atom or a nitro group, and the electron-withdrawing groups which form a cyclic system together with the nitrogen atom are represented by the formulas:

[I] 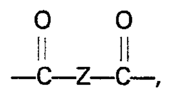

in which Z represents an alkenylene group which may be substituted by one or more halogen atoms or alkyl groups, or an arylene group which may be substituted by one or more halogen atoms,

[II] 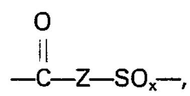

wherein x is O or 2, and Z represents an alkylene or arylene group.

3. Process according to claim 1, wherein the electron-withdrawing groups are represented by the formulas:

[I] 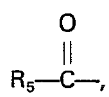

in which $R_5$ represents a dihalomethyl or trihalomethyl group, or a phenyl or naphthyl group each of which may be substituted by a methoxy group,

[II] 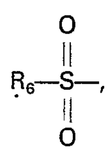

in which $R_6$ represents a methyl group, a phenyl group which may be substituted by a methyl group or a chlorine atom, an amino group, a dialkylamino group or a trialkylsilyl amino group,

[III] 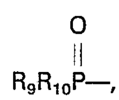

in which $R_9$ and $R_{10}$ represent a methoxy, ethoxy or propoxy group or a phenyl group which may be substituted by a nitro group or a chlorine atom, and the electron-withdrawing groups which form a cyclic system together with the nitrogen atom are represented by the formulas:

[I] 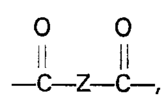

in which Z represents an ethenylene group, phenylene or naphthylene group each of which is optionally perhalo substituted,

[II]

$$-\overset{\overset{\displaystyle O}{\|}}{C}-Z-SO_x-,$$

wherein x is 0 or 2, and Z represents a phenylene group.

4. Process according to claim 1, wherein the catalyst is selected from amides, sulphonamides, cyclic or open chain imides, cyclic or open chain sulphonimides, sulphamides, disulphonamides, acylphosphoramidates, sulphonylphosphoramidates, and imidophosphates.

5. Process according to claim 4, wherein the catalyst is selected from trichloroacetamide, trifluoroacetamide, phthalimide, 3,4,5,6 - tetrachlorophthalimide, 3,4,5,6 - tetrabromophthalimide, 1,8 - naphthalimide, maleimide, barbituric acid, saccharin, N - benzoyl - 4 - toluenesulphonamide, N - (2 - methoxybenzoyl) - 4 - toluenesulphonamide, N - (1 - naphthoyl) - 4 - toluenesulphonamide, N - benzoylbenzenesulphonamide, N - (2 - methoxy - 1 - naphthoyl) - 4 - toluenesulphonamide, N - (2 - methoxy - 1 - naphthoyl)methanesulphonamide, di(4 - toluenesulphonyl)amine, dimethyl N - (trichloroacetyl)phosphoramidate, di - 4 - nitrophenyl N - (trichloroacetyl)phosphoramidate, di - 4 - nitrophenyl N - (p - toluenesulphonyl)phosphoramidate, diisopropyl N - (dichloroacetyl) - phosphoramidate, di - o - chlorophenyl N - (4 - chlorophenylsulphonyl)phosphoramidate, tetraphenyl imidodiphosphate, sulphamide, N,N - dimethylsulphamide, N,N' - bis(trimethylsilyl)sulphamide, 1,2 - benzisothiazol - 3(2H) - one and 4 - benzoyloxy - 1,2 - dihydro - 1 - oxo - phthalazine.

6. Process according to claim 5, wherein the catalyst is selected from saccharin, di - 4 - nitrophenyl N - (trichloroacetyl)phosphoramidate, di - 4 - nitrophenyl N - (4 - toluenesulphonyl)phosphoramidate and tetraphenyl imidodiphosphate.

7. Process according to claim 1 for the preparation of compounds of the general formula

$$R-S-SiMe_3$$

in which formula R represents a five- or six-membered heterocyclic group having one or more nitrogen or sulphur atoms as the hetero atoms, which group may be substituted by one or more alkyl groups, a phenyl group, a trimethylsilyl group attached to a ring nitrogen atom, an alkylamino group or a trimethylsilyloxycarbonylmethyl group.

8. Process according to claim 7, in which the group R represents a 1,3,4 - thiadiazolyl group, a 1,2,3,4 - tetrazolyl group, a 1,2,3 - triazolyl group, a 1,2,4 - triazolyl group, an imidazolyl group or a pyrimidyl group, which may be substituted by a methyl group, a phenyl group, a methylamino group, a trimethylsilyl group attached to a ring nitrogen atom or a trimethylsilyloxycarbonylmethyl group.

9. Process according to claim 7, in which the compound is selected from 2 - trimethylsilylthio - 5 - methyl - 1,3,4 - thiadiazole, 1 - methyl - 5 - trimethylsilylthio - 1H - tetrazole, 1 - trimethylsilyl - 5 - trimethylsilylthio - 1H - 1,2,3 - triazole, 1 - methyl - 2 - (trimethylsilylthio)imidazole, 1 - trimethylsilyl - 3 - trimethylsilylthio - 1H - 1,2,4 - triazole, 1 - phenyl - 5 - trimethylsilylthio - 1H - tetrazole, 4,6 - dimethyl - 2 - (trimethylsilylthio)pyrimidine, 2 - methylamino - 5 - trimethylsilylthio - 1,3,4 - thiadiazole, trimethylsilyl 5 - trimethylsilylthio - 1H - tetrazolyl - 1 - acetate, trimethylsilyl 5 - trimethylsilylthio - 1,3,4 - thiadiazolyl - 2 - acetate.

10. Process according to claim 1, wherein the catalyst is added in the form of a derivative, which derivative decomposes in the reaction mixture to give a catalyst of claim 1.

11. Process according to claim 10, wherein the derivative of the catalyst added is a silylated derivative.

**Revendications**

1. Procédé de triméthylsilylation de composés organiques portant un ou plusieurs atomes d'hydrogène actif au moyen d'hexaméthyldisilazane, caractérisé en ce que la triméthylsilylation est effectuée en présence de 0,001 à 10 moles % d'un catalyseur de la formula

$$X-NH-Y$$

où

a) X et Y sont identiques ou différents et représentent chacun un radical attirant les électrons choisi entre

$$R_1-\overset{\overset{\displaystyle O}{\|}}{C}-, \qquad R_1-\underset{\underset{\displaystyle O}{\|}}{\overset{\overset{\displaystyle O}{\|}}{S}}- \quad \text{et} \quad R_1R_2\overset{\overset{\displaystyle O}{\|}}{P}-,$$

où $R_1$ et $R_2$ sont identiques ou différents et représentent chacun un radical alcoyle qui peut être substitué par un ou plusieurs atomes d'halogène, un radical aryle qui peut être substitué par un ou plusieurs atomes d'halogène ou radicaux alcoyle, alcoxy ou nitro, un radical alcoxy, un radical aryloxy qui peut être substitué

par un atome d'halogène ou radical alcoyle ou nitro, ou un radical $R_3R_4N—$ où $R_3$ et $R_4$ sont identiques ou différents et représentent chacun un atome d'hydrogène, un radical trialcoylsilyle ou un radical alcoyle,

b) X représente un radical attirant les électrons tel que défini sous a) Y représente un atome d'hydrogène ou un radical trialcoylsilyle, ou

c) X et Y représentent ensemble un radical attirant les électrons —A—Z—B— formant un système cyclique avec l'atome d'azote, où A représente un radical

$$-\overset{\overset{O}{\|}}{C}-,$$

B représente un radical

$$-\overset{\overset{O}{\|}}{C}-,\quad -SO_2-,\quad -S-,\quad -\overset{\overset{O}{\|}}{C}-NH-\overset{\overset{O}{\|}}{C}-,\quad \text{ou}\quad -C(O\overset{\overset{O}{\|}}{C}C_6H_5)=N-,$$

et Z représente un radical alcoylène, alcénylène ou arylène, lesquels radicaux peuvent être substitués par un ou plusieurs atomes d'halogène ou radicaux alcoyle.

2. Procédé suivant la revendication 1, dans lequel les radicaux attirant les électrons sont représentés par les formules:

[I] $$R_5-\overset{\overset{O}{\|}}{C}-$$

où $R_5$ représente un radical alcoyle qui peut être substitué par un ou plusieurs atomes d'halogène, ou un radical aryle qui peut être substitué par un ou plusieurs radicaux alcoxy ou nitro,

[II] $$R_6-\underset{\underset{O}{\|}}{\overset{\overset{O}{\|}}{S}}-$$

où $R_6$ représente un radical méthyle ou un radical aryle qui peut être substitué par un ou plusieurs atomes d'halogène ou radicaux méthyle, ou bien $R_6$ représente un radical $R_7R_8N—$, où $R_7$ et $R_8$ sont identiques ou différents et représentent chacun un atome d'hydrogène, un radical trialcoylsilyle ou un radical alcoyle,

[III] $$R_9R_{10}\overset{\overset{O}{\|}}{P}-$$

où $R_9$ et $R_{10}$ sont identiques ou différents et représentent chacun un radical alcoxy ou un radical aryloxy qui peut être substitué par un atome d'halogène ou radical nitro, et les radicaux attirant les électrons qui forment un système cyclique conjointement avec l'atome d'azote sont représentés par les formules:

[I] $$-\overset{\overset{O}{\|}}{C}-Z-\overset{\overset{O}{\|}}{C}-,$$

où Z représente un radical alcénylène qui peut être substitué par un ou plusieurs atomes d'halogène ou radicaux alcoyle, ou un radical arylène qui peut être substitué par un ou plusieurs atomes d'halogène,

[II] $$-\overset{\overset{O}{\|}}{C}-Z-SO_x-,$$

où x représente 0 ou 2, et Z représente un radical alcoylène ou arylène.

3. Procédé suivant la revendication 1, dans lequel les radicaux attirant les électrons sont représentés par les formules:

[I] $$R_5-\overset{\overset{O}{\|}}{C}-,$$

où $R_5$ représente un radical dihalométhyle ou trihalométhyle ou un radical phényle ou naphtyle dont chacun peut être substitué par un radical méthoxy,

[II] 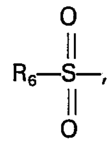

où $R_6$ représente un radical méthyle, un radical phényle qui peut être substitué par un radical méthyle ou atome de chlore, un radical amino, un radical dialcoylamino ou un radical trialcoylsilylamino,

[III] 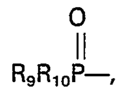

où $R_9$ et $R_{10}$ représentent un radical méthoxy, éthoxy ou propoxy ou un radical phényle qui peut être substitué par un radical nitro ou atome de chlore, et les radicaux attirant les électrons qui forment un système cyclique conjointement avec l'atome d'azote sont représentés par les formules:

[I] 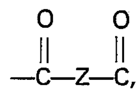

où Z représente un radical éthénylène, phénylène ou naphtylène dont chacun est éventuellement perhalosubstitué,

[II] 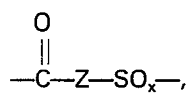

où x représente 0 ou 2 et Z représente un radical phénylène.

4. Procédé suivant la revendication 1, dans lequel le catalyseur est choisi parmi les amides, les sulfonamides, les imides cycliques ou en chaîne ouverte, les sulfonimides cycliques ou en chaîne ouverte, les sulfamides, les disulfonamides, les acylphosphoramidates, les sulfonylphosphoramidates et les imidodiphosphates.

5. Procédé suivant la revendication 4, dans lequel le catalyseur est choisi parmi le trichloroacétamide, le trifluoroacétamide, le phtalimide, le 3,4,5,6-tétrachlorophtalimide, le 3,4,5,6-tétrabromophtalimide, le 1,8-naphtalimide, le maléimide, l'acide barbiturique, la saccharine, le N - benzoyl - 4 - toluènesulfonamide, le N - (2 - méthoxybenzoyl) - 4 - toluènesulfonamide, l N - (1 - naphtoyl) - 4 - toluènesulfonamide, le N - benzoylbenzènesulfonamide, le N - (2 - méthoxy - 1 - naphtoyl) - 4 - toluènesulfonamide, le N - (2 - méthoxy - 1 - naphtoyl)méthanesulfonamide, la di - (4 - toluènesulfonyl)amine, le N - (trichloroacétyl)phosphoramidate de diméthyle, le N - (trichloroacétyl)phosphoramidate de di - 4 - nitrophényle, le N - (p - toluènesulfonyl)phosphoramidate de di - 4 - nitrophényle, le N - (dichloroacétyl)phosphoramidate de diisopropyle, le N - (4 - chlorophénylsulfonyl)phosphoramidate de di - o - chlorophényle, l'imidodiphosphate de tétraphényle, le sulfamide, le N,N - diméthylsulfamide, le N,N' - bis(triméthylsilyl)sulfamide, la 1,2-benzisothiazol - 3(2H) - one et la 4 - benzoyloxy - 1,2 - dihydro - 1 - oxo - phtalazine.

6. Procédé suivant la revendication 5, dans lequel le catalyseur est choisi parmi la saccharine, le N - (trichloroacétyl)phosphoramidate de di - 4 - nitrophényle, le N - (4 - toluènesulfonylphosphoramidate de di - 4 - nitrophényle et l'imidodiphosphate de tétraphényle.

7. Procédé suivant la revendication 1 de préparation de composés de la formule générale

$$R—S—SiMe_3$$

dans laquelle formule R représente un radical hétérocyclique à cinq ou six chaînons comprenant un ou plusieurs atomes d'azote ou de soufre comme hétéroatomes, lequel radical peut être substitué par un ou plusieurs radicaux alcoyle, un radical phényle, un radical triméthylsilyle uni à un atome d'azote du cycle, un radical alcoylamino ou un radical triméthylsilyloxycarbonylméthyle.

8. Procédé suivant la revendication 7, dans lequel le radical R représente un radical 1,3,4-thiadiazolyle, un radical 1,2,3,4-tétrazolyle, un radical 1,2,3-triazolyle, un radical 1,2,4-triazolyle, un radical imidazolyle ou un radical pyrimidyle, qui peuvent être substitués par un radical méthyle, un radical phényle, un radical méthylamino, un radical triméthylsilyle uni à un atome d'azote du cycle ou un radical triméthylsilyloxycarbonylméthyle.

9. Procédé suivant la revendication 7, dans lequel le composé est choisi parmi le 2 - triméthylsilylthio -

5 - méthyl - 1,3,4 - thiadiazole, le 1 - méthyl - 5 - triméthylsilylthio - 1H - tétrazole, le 1 - triméthylsilyl - 5 - triméthylsilylthio - 1H - 1,2,3 - triazole, le 1 - méthyl - 2 - (triméthylsilylthio)imidazole, le 1 - triméthylsilyl - 3 - triméthylsilylthio - 1H - 1,2,4 - triazole, le 1 - phényl - 5 - triméthylsilylthio - 1H - tétrazole, la 4,6 - diméthyl - 2 - (triméthylsilylthio)pyrimidine, le 2 - méthylamino - 5 - triméthylsilylthio - 1,3,4 - thiadiazole, le 5 - triméthylsilylthio - 1H - tétrazolyl - 1 - acétate de triméthylsilyle et le 5 - triméthylsilylthio - 1,3,4 - thiadiazolyl - 2 - acétate de triméthylsilyle.

10. Procédé suivant la revendication 1 dans lequel le catalyseur est ajouté sous la forme d'un dérivé, lequel dérivé se décompose dans le mélange de réaction pour donner un catalyseur suivant la revendication 1.

11. Procédé suivant la revendication 10, dans lequel le dérivé du catalyseur ajouté est un dérivé silylé.

**Patentansprüche**

1. Verfahren zur Trimethylsilylierung von organischen Verbindungen, die ein oder mehrere aktive Wasserstoffatome aufweisen, mit Hexamethyldisilazan, dadurch gekennzeichnet, daß die Trimethylsilylierung in Gegenwart von 0,001 bis 10 Mol-% eines Katalysators der Formel

X—NH—Y

durchgeführt wird, in der

a) X und Y gleich oder verschieden sind und jeweils eine elektronenziehende Gruppe darstellen, ausgewählt aus

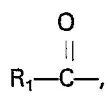, 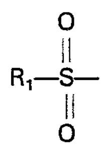 und 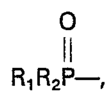,

in welchen $R_1$ und $R_2$ gleich oder verschieden sind und jeweils eine Alkylgruppe, die durch ein oder mehrere Halogenatome substituiert sein kann, eine Arylgruppe, die durch ein oder mehrere Halogenatome, Alkyl-, Alkoxy- oder Nitrogruppen substituiert sein kann, eine Alkoxygruppe, eine Aryloxygruppe, die durch ein Halogenatom, eine Alkylgruppe oder eine Nitrogruppe substituiert sein kann, oder eine Gruppe $R_3R_4N$, in der $R_3$ und $R_4$ gleich oder verschieden sind und jeweils ein Wasserstoffatom, eine Trialkylsilylgruppe oder eine Alkylgruppe bedeuten, darstellen,

b) X eine elektronenziehende Gruppe, wie unter a) definiert, und Y ein Wasserstoffatom oder eine Trialkylsilylgruppe ist, oder

c) X und Y zusammen eine elektronenziehende, mit dem Stickstoffatom ein cyclisches System bildende Gruppe —A—Z—B— darstellen, in der A eine Gruppe

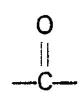

bedeutet,

B eine Gruppe

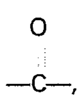, $—SO_2—$, —S—, 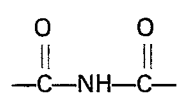

oder eine Gruppe

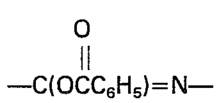

ist und Z eine Alkylen-, Alkenylen- oder Arylengruppe bedeutet, welche Gruppen durch ein oder mehrere Halogenatome oder Alkylgruppen substituiert sein können.

2. Verfahren nach Anspruch 1, dadurch gekennzeichnet, wobei die elektronenziehenden Gruppen wiedergegeben sind durch die Formeln:

[I] 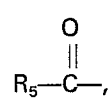,

in der $R_5$ eine Alkylgruppe, die durch ein oder mehrere Halogenatome substituiert sein kann, oder eine Arylgruppe, die durch eine oder mehrere Alkoxy- oder Nitrogruppen substituiert sein kann, darstellt,

[II] 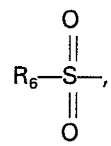

in der $R_6$ eine Methylgruppe oder eine Arylgruppe, die durch ein oder mehrere Halogenatome oder Methylgruppen substituiert sein kann, darstellt oder $R_6$ eine Gruppe $R_7R_8N—$ bedeutet, in der $R_7$ und $R_8$ gleich oder verschieden sind und jeweils ein Wasserstoffatom, eine Trialkylsilylgruppe oder eine Alkylgruppe darstellen,

[III] 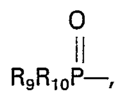

in der $R_9$ und $R_{10}$ gleich oder verschieden sind und jeweils eine Alkoxygruppe oder eine Aryloxygruppe, die durch ein Halogenatom oder eine Nitrogruppe substituiert sein kann, bedeuten, und die elektronenziehenden Gruppen, die zusammen mit dem Stickstoffatom ein cyclisches System bilden, wiedergegeben sind durch die Formeln:

[I] 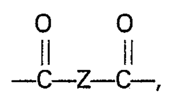

in der Z eine Alkenylengruppe, die durch ein oder mehrere Halogenatome oder Alkylgruppen substituiert sein kann, oder eine Arylengruppe, die durch ein oder mehrere Halogenatome substituiert sein kann, ist,

[II] 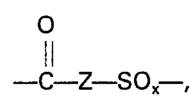

in der x 0 oder 2 ist und Z eine Alkylen- oder Arylengruppe bedeutet.

3. Verfahren nach Anspruch 1, wobei die elektronenziehenden Gruppen wiedergegeben sind durch die Formeln:

[I] 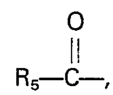

in der $R_5$ eine Diahlogenmethyl- oder Triahlogenmethylgruppe, oder eine Phenyl- oder Naphthylgruppe, von welchen jede durch eine Methoxygruppe substituiert sein kann, bedeutet,

[II] 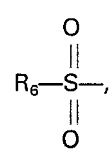

in der $R_6$ eine Methylgruppe, eine Phenylgruppe, die durch eine Methylgruppe oder ein Chloratom substituiert sein kann, eine Aminogruppe, eine Dialkylaminogruppe oder eine Trialkylsilylaminogruppe bedeutet,

[III] 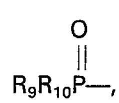

in der $R_9$ und $R_{10}$ eine Methoxy-, Ethoxy- oder Propoxygruppe oder eine Phenylgruppe, die durch eine Nitrogruppe oder ein Chloratom substituiert sein kann, darstellen, und die elektronenziehenden Gruppen, die zusammen mit dem Stickstoffatom ein cyclisches System bilden, wiedergegeben sind durch die Formeln:

[I] 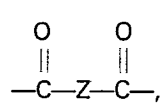

in der Z eine Ethenylengruppe, Phenylen- oder Naphthylengruppe ist, von welchen jede gewünschtenfalls Perhalogen- substituiert ist,

[II] $$-\overset{\displaystyle O}{\overset{\|}{C}}-Z-SO_x-,$$

in der x 0 oder 2 ist und Z eine Phenylengruppe darstellt.

4. Verfahren nach Anspruch 1, wobei der Katalysator ausgewählt ist aus Amiden, Sulfonamiden, cyclischen oder offenkettigen Imiden, cyclischen oder offenkettigen Sulfonimiden, Sulfamiden, Disulfonamiden, Acylphosphoramidaten, Sulfonylphosphoramidaten und Imidophosphaten.

5. Verfahren nach Anspruch 4, wobei der Katalysator ausgewählt ist aus Trichloracetamid, Trifluoracetamid, Phthalimid, 3,4,5,6-Tetrachlorphthalimid, 3,4,5,6-Tetrabromphthalimid, 1,8-Naphthalimid, Maleinimid, Barbitursäure, Saccharin, N-Benzoyl - 4 - toluolsulfonamid, N - (2 - Methoxybenzoyl) - 4 - toluolsulfonamid, N - (1 - Naphthoyl) - 4 - toluolsulfonamid, N - Benzoylbenzolsulfonamid, N - (2 - Methoxy - 1 - naphthoyl) - 4 - toluolsulfonamid, N - (2 - Methoxy - 1 - naphthoyl) - methansulfonamid, Di(4 - toluolsulfonyl)amin, Dimethyl - N - (trichloracetyl)phosphoramidat, Di - 4 - nitrophenyl - N - (trichloroacetyl)phosphoramidat, Di - 4 - nitrophenyl - N - (p - toluolsulfonyl)phosphoramidat, Diisopropyl - N - (dichloroacetyl)phosphoramidat, Di - o - chlorophenyl - N - (4 - chlorphenylsulfonyl)phosphoramidat, Tetraphenyl - imidodiphosphat, Sulfamid, N,N-Dimethylsulfamid, N,N'-Bis(trimethylsilyl)sulfamid, 1,2 - Benzisothiazol - 3(2H) - on und 4 - Benzoyloxy - 1,2 - dihydro - 1 - oxo - phthalazin.

6. Verfahren nach Anspruch 5, wobei der Katalysator ausgewählt ist aus Saccharin, Di - 4 - nitrophenyl - N - (trichloroacetyl)phosphoramidat, Di - 4 - nitrophenyl - N - (4 - toluolsulfonyl)-phosphoramidat und Tetraphenyl-imidodiphosphat.

7. Verfahren nach Anspruch 1 für die Herstellung von Verbindungen der allgemeinen Formel

$$R-S-SiMe_3,$$

in welcher Formel R eine 5- oder 6-gliedrige heterocyclische Gruppe mit einem oder mehreren Stickstoff- oder Schwefelatomen als Heteroatome, welche Gruppe durch eine oder mehrere Alkylgruppen, eine Phenylgruppe, eine an ein Ringstickstoffatom gebundene Trimethylsilylgruppe, eine Alkylaminogruppe oder eine Trimethylsilyloxycarbonylmethylgruppe substituiert sein kann, darstellt.

8. Verfahren nach Anspruch 7, bei welchem die Gruppe R eine 1,3,4-Thiadiazolylgruppe, eine 1,2,3,4-Tetrazolylgruppe, eine 1,2,3-Triazolylgruppe, eine 1,2,4-Triazolylgruppe, eine Imidazolylgruppe oder Pyrimidylgruppe, die durch eine Methylgruppe, eine Phenylgruppe, eine Methylaminogruppe, eine an ein Ringstickstoffatom gebundene Trimethylsilylgruppe oder eine Trimethylsilyloxycarbonylmethylgruppe substituiert sein können, darstellt.

9. Verfahren nach Anspruch 7, bei welchem die Verbindung ausgewählt ist aus 2 - Trimethylsilylthio - 5 - methyl - 1,3,4 - thiadiazol, 1 - Methyl - 5 - trimethylsilylthio - 1H - tetrazol, 1-Trimethylsilyl - 5 - trimethylsilylthio - 1H - 1,2,3 - triazol, 1 - Methyl - 2 - (trimethylsilylthio)imidazol, 1 - Trimethylsilyl - 3 - trimethylsilylthio - 1H - 1,2,4 - triazol, 1 - Phenyl - 5 - trimethylsilylthio - 1H - tetrazol, 4,6 - Dimethyl - 2 - (trimethylsilylthio)pyrimidin, 2 - Methylamino - 5 - trimethylsilylthio - 1,3,4 - thiadiazol, Trimethylsilyl - 5 - trimethylsilylthio - 1H - tetrazolyl - 1 - acetat, Trimethylsilyl - 5 - trimethylsilylthio - 1,3,4 - thiadiazolyl - 2 - acetat.

10. Verfahren nach Anspruch 1, wobei der Katalysator in Form eines Derivats zugesetzt wird, welches Derivat sich in der Reaktionsmischung zersetzt und dabei einen Katalysator gemäß Anspruch 1 ergibt.

11. Verfahren nach Anspruch 10, wobei das zugefügte Derivat des Katalysators ein silyliertes Derivat ist.